(19) Europäisches Patentamt
European Patent Office
Office européen des brevets

(11) Veröffentlichungsnummer: **0 388 682**
**A1**

# (12) EUROPÄISCHE PATENTANMELDUNG

(21) Anmeldenummer: **90104059.2**

(22) Anmeldetag: **02.03.90**

(51) Int. Cl.[5]: **C07D 213/65, C07D 417/12, C07D 213/73, C07D 213/64, C07D 241/18, C07D 277/68, C07D 239/34, C07D 401/12, C07D 213/80, C07D 307/81, C07D 409/12, //C07D405/12, C07D213/68,C07D213/70, C07D213/30,A01N43/40, A01N43/54,A01N43/60, A01N43/74,A01N43/12**

(30) Priorität: **15.03.89 DE 3908452**
**07.04.89 DE 3911217**
**24.10.89 DE 3935287**

(43) Veröffentlichungstag der Anmeldung:
**26.09.90 Patentblatt 90/39**

(84) Benannte Vertragsstaaten:
**AT BE CH DE DK ES FR GB IT LI NL**

(71) Anmelder: **BAYER AG**
**D-5090 Leverkusen 1 Bayerwerk(DE)**

(72) Erfinder: **Alig, Bernd, Dr.**
**Gartenstrasse 2**
**D-5330 Königswinter 21(DE)**
Erfinder: **Stendel, Wilhelm, Dr.**
**In den Birken 55**
**D-5600 Wuppertal 1(DE)**
Erfinder: **Londershausen, Michael, Dr.**
**Galileistrasse 11**
**D-4006 Erkrath 2(DE)**

## (54) Substituierte Heteroarylphenylether, Verfahren zu ihrer Herstellung und ihre Verwendung als Insektizide.

(57) Die vorliegende Erfindung betrifft neue substituierte Heteroarylphenylether der Formel (I)

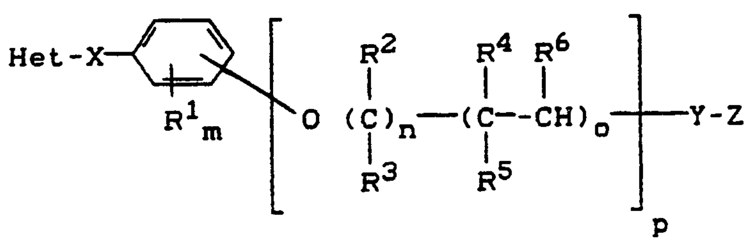

(I)

in welcher
Het für einen gegebenenfalls substituierten heteroaromatischen Rest steht,
X für O, S, $-CH_2-$, $-O-CH_2-$, $-S-CH_2-$,
$-\overset{O}{\overset{\|}{C}}-$ steht;
Y für O, $-O-CH_2-$, S steht,
Z für einen gegebenenfalls substituierten aromatischen oder heteroaromatischen Rest steht;

EP 0 388 682 A1

m, n, o, p unabhängig voneinander für ganze Zahlen von 1 bis 4 stehen,
$R^2$, $R^3$, $R^4$, $R^5$, $R^6$ unabhängig voneinander für Wasserstoff, $C_1$-$C_6$-Alkyl, des gegebenenfalls durch Halogen oder $C_{1-4}$-Alkoxy substituiert ist, stehen; zwei benachbart voneinander stehende Reste können auch gemeinsam mit den C-Atomen, an die sie gebunden sind, einen gesättigten carbocyclischen 5- oder 6-Ring bilden,
$R^1$ für gleiche oder verschiedene Reste aus der Gruppe Wasserstoff, Halogen, CN, $C_{1-4}$-Alkyl, 1-5-Halogen-$C_{1-4}$-alkyl, $C_{1-4}$-Alkoxy, $C_{1-4}$-Alkylthio, 1-5-Halogen-$C_{1-4}$-alkoxy, 1-5-Halogen-$C_{1-4}$-alkylthio, Phenyl, Phenoxy steht,
wobei die Verbindungen der Formel I gegebenenfalls in Form ihrer Enantiomere, Racemate oder Diastereomere vorliegen können, sowie Verfahren zu ihrer Herstellung und ihre Verwendung als Insektizide.

## Substituierte Heteroarylphenylether, Verfahren zu ihrer Herstellung und ihre Verwendung als Insektizide

Die vorliegende Erfindung betrifft substituierte Heteroarylphenylether, Verfahren zu ihrer Herstellung und ihre Verwendung als Insektizide.

Substituierte Diphenylether und ihre Verwendung als Insektizide sind bereits bekannt geworden. Ihre Wirkung vor allem gegen die verschiedenen Entwicklungsstadien von Flöhen befriedigt jedoch nicht in jedem Fall (DE-PS 2 520 145, EP-OS 128 648).

Gegenstand der vorliegenden Erfindung sind

1. Neue substituierte Heteroarylphenylether der Formel (I)

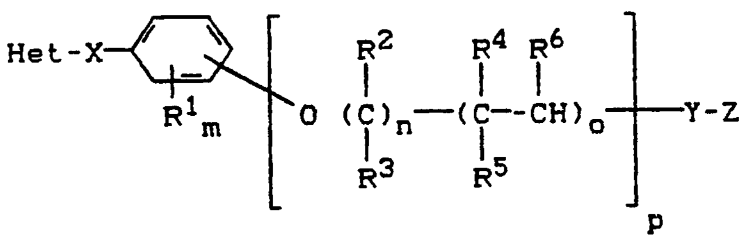

(I)

in welcher

Het für einen gegebenenfalls substituierten heteroaromatischen Rest steht,

X für O, S, $-CH_2-$, $-O-CH_2-$, $-S-CH_2-$, $-CH_2-O-$,

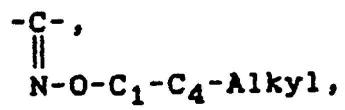

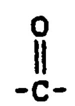 steht;

Y für O, $-O-CH_2-$, S steht,

Z für einen gegebenenfalls substituierten aromatischen oder heteroaromatischen Rest steht;

m für ganze Zahlen von 1 bis 4 steht

n für 0, 1, 2, 3, 4 steht,

o für 0, 1, 2, 3, 4 steht,

wobei n und o nicht gleichzeitig für 0 stehen dürfen,

p für ganze Zahlen von 1 bis 4 steht,

$R^2$, $R^3$, $R^4$, $R^5$, $R^6$ unabhängig voneinander für Wasserstoff, $C_1$-$C_6$-Alkyl, das gegebenenfalls durch Halogen oder $C_{1-4}$-Alkoxy substituiert ist, stehen; zwei benachbart voneinander stehende Reste können auch gemeinsam mit den C-Atomen, an die sie gebunden sind, einen gesättigten carbocyclischen 5- oder 6-Ring bilden,

$R^1$ für gleiche oder verschiedene Reste aus der Gruppe Wasserstoff, Halogen, CN, $C_{1-4}$-Alkyl, 1-5-Halogen-$C_{1-4}$-alkyl, $C_{1-4}$-Alkoxy, $C_{1-4}$-Alkylthio, 1-5-Halogen-$C_{1-4}$-alkoxy, 1-5-Halogen-$C_{1-4}$-alkylthio, Phenyl, Phenoxy steht,

wobei die Verbindungen der Formel I gegebenenfalls in Form ihrer Enantiomere, Racemate oder Diastereomere vorliegen können.

2. Verfahren zur Herstellung der substituierten Heteroarylphenylether der Formel (I)

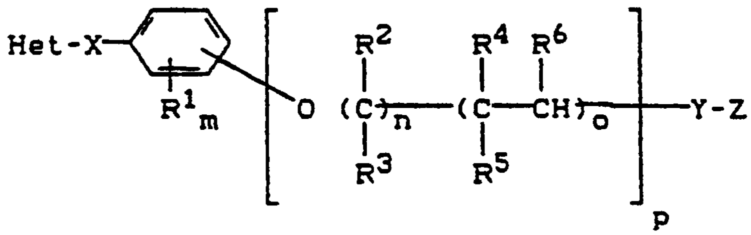

(I)

dadurch gekennzeichnet, daß man

a) Heteroarylphenolether der Formel (II)

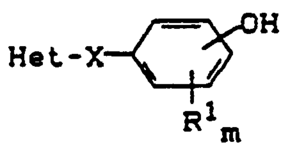

(II)

in welcher
Het, X, $R^1$, m die oben angegebene Bedeutung haben,
mit Verbindungen der Formel (III)

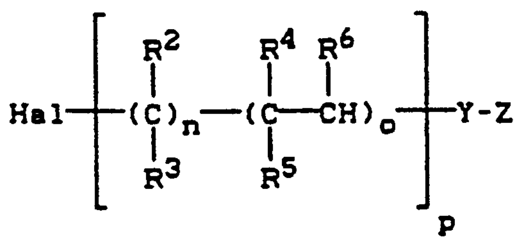

(III)

in welcher
Hal für Halogen steht und
Y, Z, $R^2$ - $R^6$, n, o, p die oben angegebene Bedeutung haben,
umsetzt oder

b) Verbindungen der Formel (IV)

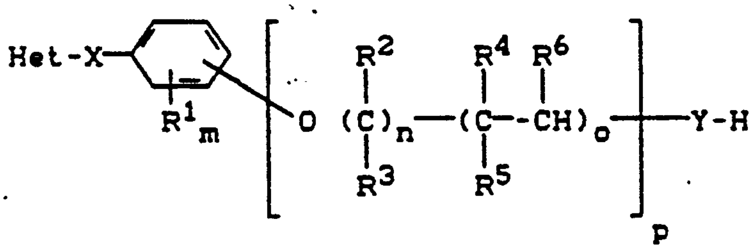

(IV)

in welcher
X, Het, $R^1$ - $R^6$, m, n, o, p die oben angegebene Bedeutung haben und
Y für O oder S steht;
mit Verbindungen der Formel (V)

Hal-Z oder Hal-$CH_2$-Z (V)

in welcher
Hal für Halogen steht,
Z die oben angegebene Bedeutung hat,
umsetzt oder

c) Verbindungen der Formel (VI)

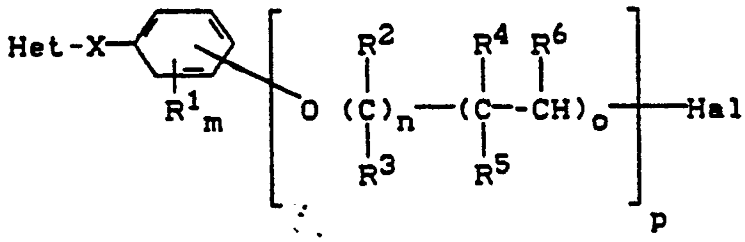

(VI)

in welcher
Hal für Halogen steht,
X, Het, $R^1$ - $R^6$, m, n, o, p die oben angegebene Bedeutung haben,
mit Verbindungen der Formel (VII)
H-Y-Z (VII)
in welcher
Y und Z die oben angegebene Bedeutung haben,
umsetzt oder

d) für den Fall, daß Het und Z für identische Heterocyclen stehen, man Verbindungen der Formel (VIII)

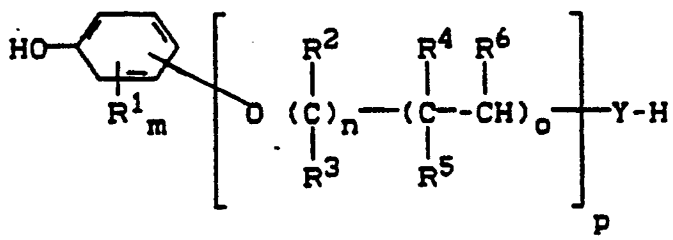

(VIII)

in welcher
$R^1$ - $R^6$, m, n, o, p die oben angegebene Bedeutung haben und
Y für O, S steht,
mit heterocyclischen Verbindungen der Formel (IX)
Het-Hal oder Het-$CH_2$-Hal (IX)
in welcher
Hal für Halogen steht,
Het die oben angegebene Bedeutung hat,
umsetzt.

Die Verbindungen der Formel I sowie ihre Salze mit Säuren eignen sich zur Bekämpfung von Insekten. Sie lassen sich besonders bevorzugt zur Bekämpfung von Flöhen einsetzen, insbesondere gegen Pulex irritans, Xenopsylla cheopis, Ctenocephalides canis, Ctenocephalides felis, Ceratophyllus gallinae, Echidnophaga gallinacea, Tunga penetrans. Die Verbindungen der Formel I besitzen die Entwicklung von Insekten hemmende Eigenschaften. Sie können daher gegen alle oder einzelne Entwicklungsstadien von Insekten, insbesondere der Flöhe, eingesetzt werden.

Als heteroaromatische Reste in der Bedeutung Het oder Z seien bevorzugt die Stickstoffheterocyclen genannt Pyridyl, Pyrimidinyl, Pyrazinyl, Pyridazinyl, Quinolyl, Isoquinolyl, Phthalazinyl, Naphthyridyl, Quinoxalinyl, Quinazolinyl, Cinnolinyl, Thiazolyl, Benzthiazolyl, Oxazolyl, Benzoxazolyl, Pyrazolyl, Imidazolyl, Benzimidazolyl sowie Furyl, Thienyl, Benzofuranyl, Benzothienyl, die über ein C-Atom der Heterocyclen an den übrigen Rest der Verbindung der Formel (I) gebunden sind.

Die heteroaromatischen Reste in der Bedeutung Het oder Z können unabhängig voneinander ein- oder mehrfach substituiert sein durch Halogen, insbesondere Chlor, Brom, Fluor, $C_{1-4}$-Alkyl, 1-5-Halogen-$C_{1-4}$-alkyl, $C_{1-4}$-Alkoxy, 1-5-Halogen-$C_{1-4}$-Alkoxy, $C_{1-4}$-Alkylthio, 1-5-Halogen-$C_{1-4}$-alkylthio, Nitro, CN, $C_{1-4}$-Alkoxycarbonyl, Amino, Acylamino.

Als aromatischer Rest in der Bedeutung Z sei bevorzugt genannt Phenyl, das gegebenenfalls durch einen oder mehrere der bei Het (oben) angegebenen Substituenten substituiert sein kann.

Bevorzugt sind Verbindungen der Formel (I), in welcher: Het für gegebenenfalls substituierte stickstoffhaltige Heteroaromaten steht, die über ein C-Atom des Heterorings an den übrigen Rest der Moleküle

gebunden sind. Insbesondere seien genannt: Pyridyl, Pyrimidinyl, Pyrazinyl, Pyridazinyl, Thiazolyl, die gegebenenfalls an einen Phenylring ankondensiert sein können;

Als Substituenten seien genannt Halogen, insbesondere Fluor, Chlor, Brom, $C_{1-4}$-Alkyl, insbesondere Methyl, 1-5-Halogen-$C_{1-2}$-alkyl, insbesondere Trifluormethyl, $C_{1-4}$-Alkoxy, insbesondere Methoxy, Ethoxy, $C_{1-4}$-Alkylmercapto, insbesondere Methylmercapto, 1-3-Halogen-$C_{1-2}$-alkoxy, insbesondere Trifluormethoxy, 1-3-Halogen-$C_{1-2}$-alkylmercapto.

X für O steht;

$R^1$ für gleiche oder verschiedene Reste aus der Gruppe Wasserstoff, Halogen, insbesondere Chlor oder Fluor, CN, $C_{1-4}$-Alkyl, insbesondere Methyl, Ethyl, t-Butyl, $C_{1-4}$-Alkoxy, insbesondere Methoxy, 1-3-Halogen-$C_{1-2}$-alkoxy, insbesondere Trifluormethoxy, Phenyl steht;

m für ganze Zahlen von 1 bis 4 steht;

$R^2$, $R^3$, $R^4$, $R^5$, $R^6$ unabhängig voneinander für Wasserstoff, $C_{1-4}$-Alkyl, insbesondere Methyl, 1-3-Halo gen-$C_{1-2}$-alkyl, insbesondere Trifluormethyl, $C_{1-2}$-Alkoxy-$C_{1-4}$-alkyl, insbesondere Methoxymethyl, Ethoxymethyl, i-Propoxymethyl, t-Butoxymethyl stehen;

n, o, p unabhängig voneinander für ganze Zahlen von 1 bis 4 stehen, wobei n und o nicht gleichzeitig für 0 stehen dürfen,

p für ganze Zahlen von 1 bis 4 steht.

Insbesondere steht der folgende Teil der Formel (I)

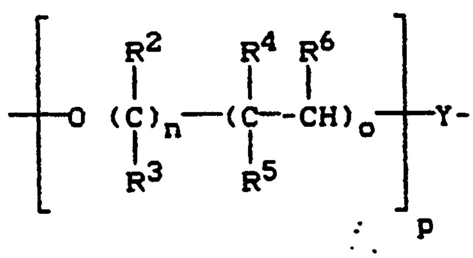

für eine der folgenden Strukturen:

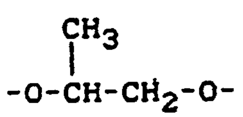 ; 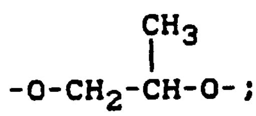 ; $-O-(CH_2)_{1-4}-O-$ ;

$-O-(CH_2)_{1-4}-S-$ ; 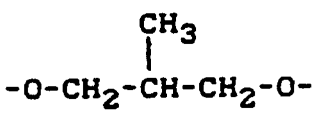 ;

$-O-CH_2-C(CH_3)_2-CH_2-O-$ ; 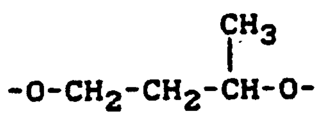 ;

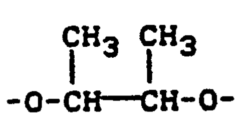 ; 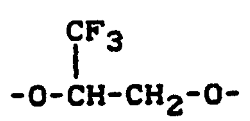 ;

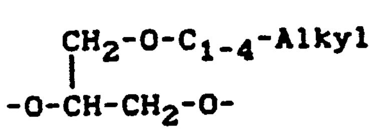 ; $-O-CH_2-CH=CH-CH_2-O-$ ;

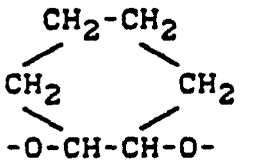 ; 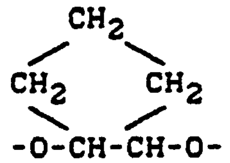 ;

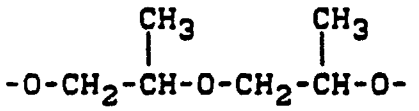 ; 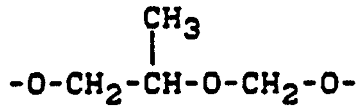 .

Z steht bevorzugt für einen der bei der bevorzugten Definition von Het angegebenen Heteroaromaten, der gegebenenfalls durch einen oder mehrere der dort angegebenen Substituenten substituiert sein kann. Weiter steht Z bevorzugt für Phenyl, das gegebenenfalls durch einen oder mehrere der bei Het angegebenen Substituenten sowie gegebenenfalls durch Methylendioxy, Ethylendioxy, Dihalogenmethylendioxy substituiert ist.

Besonders bevorzugt sind Verbindungen der Formel (I), in welcher
Het für gegebenenfalls substituiertes Pyridyl, Pyrimidinyl, Pyrazinyl, Thiazolyl, Benzthiazolyl steht, die über ein C-Atom des Heteroaromaten an den übrigen Rest der Verbindung der Formel (I) gebunden sind. Als Substituenten seien insbesondere genannt: Chlor, Brom, Methyl, Ethyl, Trifluormethyl, Methoxy.
X für O steht;
$R^1$ für Wasserstoff steht;
der folgende Teil der Formel (I)

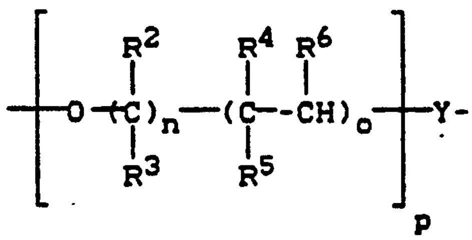

in para- oder meta-Stellung zum Rest Het-X- steht und für eine der folgenden Strukturen steht:

$-O-CH_2-CH_2-O-$ ; 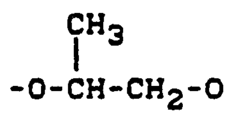 ;

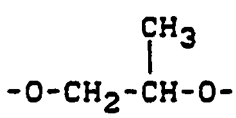 ; 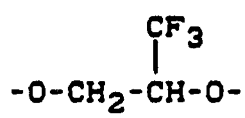 ;

$-O-CH_2-CH_2-O-CH_2-$ ; $-O-(CH_2)_3-O-$ ;

Z insbesondere für gegebenenfalls substituiertes Pyridyl, Pyrimidinyl, Pyrazinyl steht, die über ein C-Atom an den übrigen Rest der Verbindung der Formel (I) gebunden sind sowie für gegebenenfalls substituiertes Phenyl steht Als Substituenten seien genannt: Fluor, Chlor, Brom, Methyl, Trifluormethyl.

Im einzelnen seien genannt:

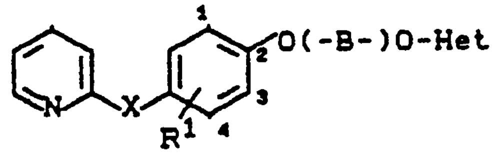

| X | $R^1$ | B | Het |
|---|---|---|---|
| O | 1,3-$Cl_2$ | $C_2H_5$<br>$-CH_2-CH-$ | 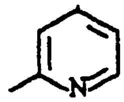 |
| S | H | $CF_3$<br>$-CH_2-CH$ | 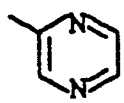 |
| O | 1,3-$Cl_2$ | $-CH_2-CH-$<br>$CH_2F$ | 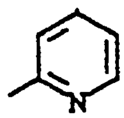 |
| O | 1,3-$Cl_2$ | $-CH_2-CH-$<br>$CH_2-F$ | 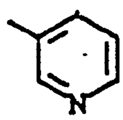 |
| S | 1,3-$Cl_2$ | $-CH_2-CH-$<br>$CH_2-F$ | 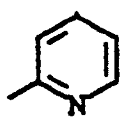 |
| O | 3-Br | $-CH_2-CH-$<br>$CH_2-F$ | 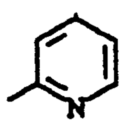 |

Le A 26 734

| X | $R^1$ | B | Het |
|---|---|---|---|
| $CH_2$ | 1,3-$Cl_2$ | 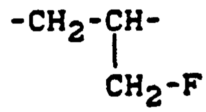 | 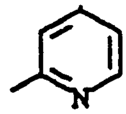 |
| $CH_2$ | H | 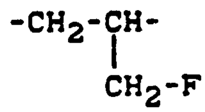 | 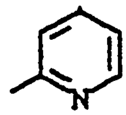 |
| O | H | 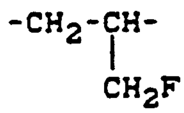 | 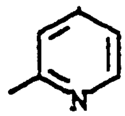 |
| S | H | 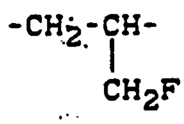 | 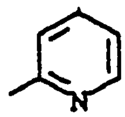 |
| O | 1,3-$Cl_2$ | 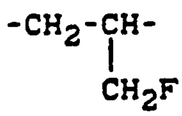 | 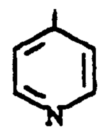 |
| O | 1,3-$Cl_2$ | 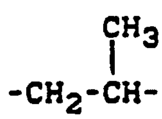 | 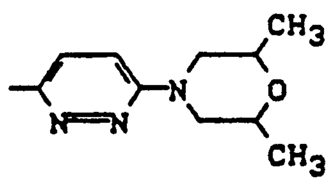 |
| S | 1,3-$Cl_2$ | 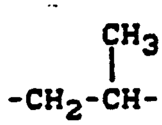 | 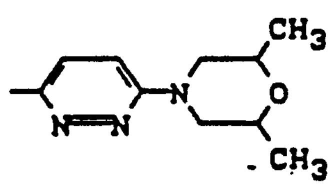 |

| X | $R^1$ | B | Het |
|---|---|---|---|
| $CH_2$ | 1,3-$Cl_2$ | $CH_3$<br>$-CH_2-CH-$ | 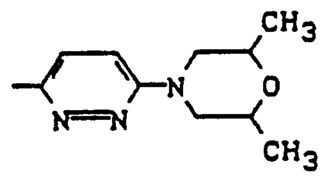 |
| O | H | $CH_3$<br>$-CH_2-CH-$ | 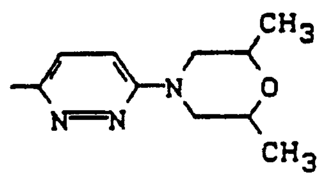 |
| O | 1,3-$Cl_2$ | $CH_3$<br>$-CH_2CH_2O-CH_2-CH_2-$ | 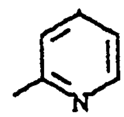 |

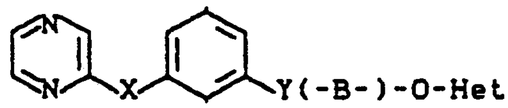

| X | Y | B | Het |
|---|---|---|---|
| O | S | $-CH_2CH_2OCH_2CH_2$ | 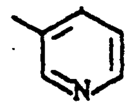 |
| O | O | $CH_2-CH_2-O-CH_2-CH_2$ | 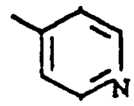 |
| O | S | $-CH_2-CH-$<br>$CH_2-F$ | 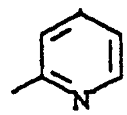 |
| S | O | $-CH_2-CH-$<br>$CH_2-F$ | 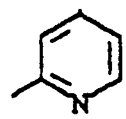 |
| O | O | $-CH_2-CH-$<br>$CH_2-F$ | 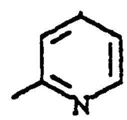 |
| $CH_2$ | S | $-CH_2-CH-$<br>$CH_2-F$ | 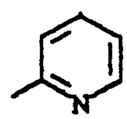 |
| O | O | $CH_3$<br>$-CH_2-CH-$ | 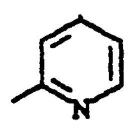 |

| X | Y | B | Het |
| --- | --- | --- | --- |
| O | O | 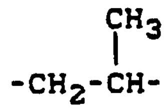 | 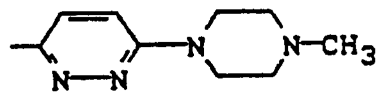 |
| O | O | 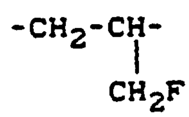 | 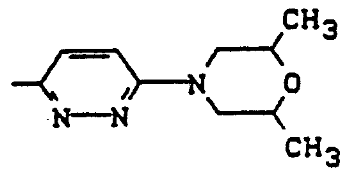 |
| S | O | 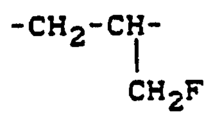 | 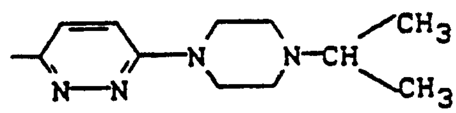 |
| $CH_2$ | O | 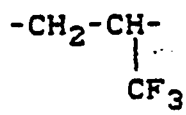 | 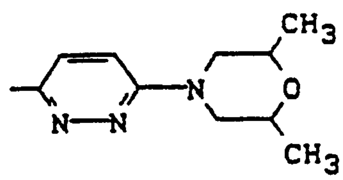 |
| O | O | 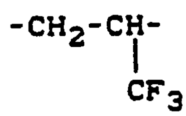 | 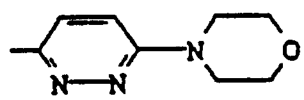 |
| O | O | 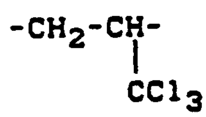 | 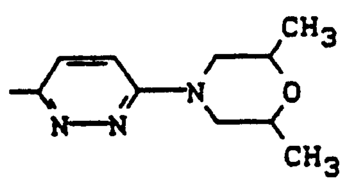 |
| S | O | 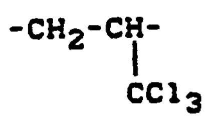 | 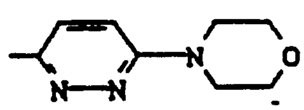 |

Setzt man bei Verfahren 2 a als Verbindung der Formel (III) 2-Chlorethoxy-methoxy-4-chlorbenzol und als Verbindung der Formel (II) 4-(4-Pyridyloxy)phenol ein, läßt sich der Reaktionsverlauf durch folgendes Reaktionsschema kennzeichnen:

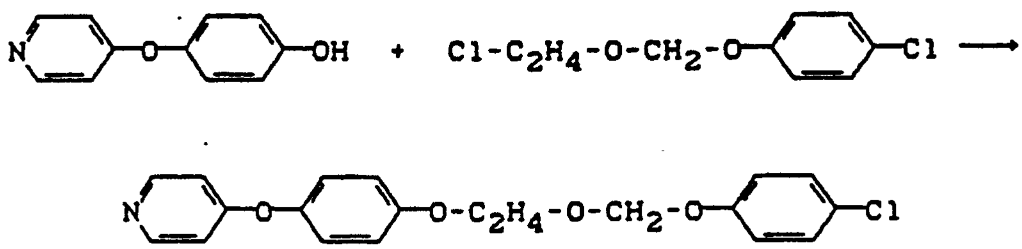

Bevorzugt werden Verbindungen der Formel (II) und (III) eingesetzt, in denen $R^1$, $R^2$, $R^3$, $R^4$, $R^5$, $R^6$, X, Y, Z, Het, m, n, o, p die bei den Verbindungen der Formel (I) angegebenen bevorzugten oder besonders bevorzugten Bedeutungen besitzen.

Im einzelnen seien folgende Verbindungen der Formel (II) genannt:

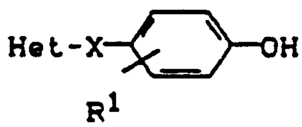

| Het | X | $R^1$ |
|---|---|---|
| 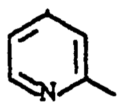 | O | H |
| 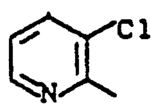 | O | H |
| 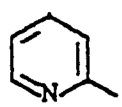 | O | 3-Cl |
| 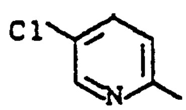 | O | H |

Im einzelnen seien folgende Verbindungen der Formel (III) genannt:

Hal-M-Y-Z

| Hal | M | Y | Z |
|---|---|---|---|
| Cl | $-CH_2CH_2-$ | O | 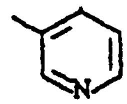 |
| Cl | $-CH_2CH_2-$ | O | 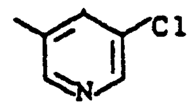 |
| Cl | $-CH_2CH_2-$ | O | 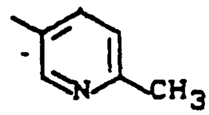 |

Fortsetzung

| Hal | M | Y | Z |
|---|---|---|---|
| Br | $-CH_2CH_2-$ | $OCH_2$ | 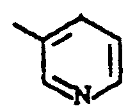 |
| Br | $-CH_2CF_2-$ | O | 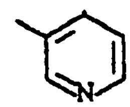 |
| Br | $-CH_2CH_2$ | O | 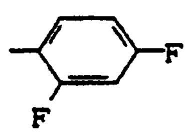 |
| Br | $-CH_2CH_2CH_2-$ | O | 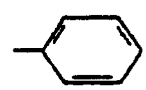 |

Die Verbindungen der Formeln (II) und (III) sind bekannt oder lassen sich analog in bekannten Verfahren herstellen.

Die Umsetzung erfolgt durch Erhitzen der Verbindungen II und III in Gegenwart von Verdünnungsmitteln und Basen.

Die Umsetzung erfolgt bei Temperaturen von 0 - 200 °C, bevorzugt bei 20 - 100 °C, besonders bevorzugt bei Siedetemperatur des Verdünnungsmittels.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Alkohole wie Methanol, Ethanol,

Isopropanol, Butanol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Als Basen kommen anorganische und organische Basen in Frage. Als Basen seien genannt Alkali- und Erdalkalihydroxide, -carbonate, -hydrogencarbonate, -alkoholate, ferner Amine wie insbesondere tertiäre Amine z.B. Trimethylamin, Triethylamin, N-Methylmorpholin, Pyridin, Picoline, N-Ethylpyrrolidin, Diazabicyclo(4,3,0)-undecan(DBU), 1,4-Diazabicyclo(2,2,2)octan (DABCO), Diazabicyclo(3,2,0)nonen (DBN).

Die Verbindungen der Formeln II und III werden in etwa äquimolarem Verhältnis zueinander eingesetzt. Ein Überschuß der einen oder anderen Komponente bringt keinen wesentlichen Vorteil.

Nach erfolgter Umsetzung wird das Verdünnungsmittel abdestilliert und die Verbindungen der Formel I in an sich bekannter Weise isoliert, indem sie z.B. aus dem Rückstand mit einem geeigneten Lösungsmittel z.B. Ether extrahiert werden. Die Verbindungen der Formel I können anschließend in üblicher Weise z.B. durch Destillation gereinigt werden.

Setzt man bei Verfahren 2b zur Herstellung der erfindungsgemäßen Verbindungen als Verbindung der Formel (IV) 1-Methyl-2-[4-(4-Pyridyloxy)phenoxy]ethanol und als Verbindung der Formel (V) 3-Chlorpyridin ein, läßt sich der Reaktionsverlauf durch folgendes Formelschema kennzeichnen:

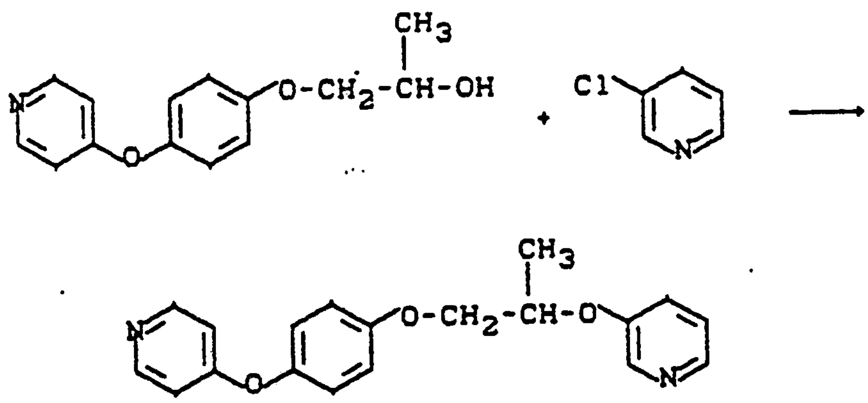

Bevorzugt werden Verbindungen der Formeln (IV) und (V) eingesetzt, in denen $R^1$, $R^2$, $R^3$, $R^4$, $R^5$, $R^6$, X, Y, Z, Het, m, n, o, p die bei den Verbindungen der Formel (I) angegebenen bevorzugten oder besonders bevorzugten Bedeutungen besitzen.

Im einzelnen seien folgende Verbindungen der Formel (IV) genannt:

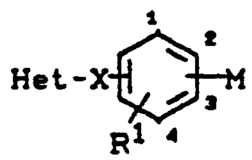

Het = 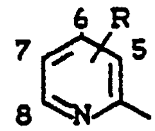

| R[Het] | X | $R^1$ | M |
|---|---|---|---|
| H | $CH_2$ | 1-Cl | 3-O-$CH_2$-$CH_2$-SH |
| 7-Cl | O | H | 2-O-$CH_2$-$CH_2$-SH |
| 7-Cl | O | H | 2-O-$CH_2$-$CH_2$-OH |
| 7-Cl | S | H | 2-O-$CH_2$-$CH_2$-OH |
| H | O | 3-$OCH_3$ | 2-O-$CH_2$-CH-OH<br>$CH_3$ |
| H | O | 3-$OCH_3$ | 2-O-$CH_2$-CH-OH<br>$CF_3$ |
| 7-Cl | CH-$CH_3$ | H | 2-O-$CH_2$-$CH_2$-SH |
| 7-Cl | O | H | 2-O-$CH_2$-CH-OH<br>$CH_2F$ |
| 7-Cl | O | H | 3-O-$CH_2$-CH-OH<br>$CH_2F$ |
| H | S | 3-$OCH_3$ | 2-O-$CH_2$-CH-OH<br>$CH_2F$ |
| 7-Cl | O | H | 3-O-$CH_2$-CH-SH<br>$CH_2$-$OCF_3$ |
| 7-Cl | S | H | 2-O-$CH_2$-CH-OH<br>$CH_2$-$OCF_3$ |
| H | O | 1-Cl,3-t-Butyl | 2-O-CH-$CH_2$-OH<br>$CH_3$ |

In einzelnen seien folgende Verbindungen der Formel V genannt: 2-Chlorpyridin, 2-Brompyridin, 2-Chlorpyrimidin, 2-Brompyrimidin, 3-Chlorpyridin, 3-Brompyridin, 2-Chlor-5-trifluormethylpyridin, 2-Brom-5-methylpyridin, 4-Chlorpyridin, 2-Chlorbenzothiazol, 2-Chlorpyrazin, 2-Bromthiazol, 2-Chlor-6-methylpyridin, 5-Brompyrimidin, 2-Bromthiophen, 3-Bromthiophen, 3-Chlor-2,5-dimethylpyrazin, 4-Chlor-2-methylthiopyrimidin, 2-Chlorthiophen, 2,3-Dichlorpyridin, 2,5-Dichlorpyridin, 2,6-Dichlorpyridin, 2,6-Dibrompyridin, 3,5-Dichlorpyridin, 2,4-Dichlor-6-methylpyrimidin, 2,4-Dichlorpyrimidin, 4,6-Dichlorpyrimidin, 2,6-Difluorpyridin, 4,6-Dichlor-2-methylthiopyrimidin, 2,6-Dichlor-pyrazin, 3,6-Dichlorpyridazin, 3-Chlor-6-N′-Alkylpiperazinyl-pyridazin, 3-Chlor-6-dimethylmorpholinyl-pyridazin, 2-Picolylchlorid, 3-Picolylchlorid, 4-Picolylchlorid.

Die Verbindungen der Formeln (IV) und (V) sind bekannt (EP-OS 3877, US-P 4 491 468).

Die Umsetzung erfolgt durch Erhitzen der Verbindungen IV und V in Gegenwart von Verdünnungsmitteln, Basen sowie gegebenenfalls Katalysatoren.

Die Umsetzung erfolgt bei Temperaturen von 0 - 200° C, bevorzugt bei 20 - 100° C, besonders bevorzugt bei Siedetemperatur des Verdünnungsmittels.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Als Basen kommen anorganische und organische Basen in Frage. Als Basen seien genannt Alkali- und Erdalkalihydroxide, -carbonate, -hydrogencarbonate, -alkoholate, ferner Amine wie insbesondere tertiäre Amine z.B. Trimethylamin, Triethylamin, N-Methylmorpholin, Pyridin, Picoline, N-Ethylpyrrolidin, Diazabicyclo(4,3,0)-undecan(DBU), 1,4-Diazabicyclo(2,2,2)octan (DABCO), Diazabicyclo(3,2,0)nonen (DBN).

Als Katalysatoren kommen z.B. in Frage Phasentransferkatalysatoren wie Tris-[2-(2-methoxyethoxy)]-ethyl-amin (TDA-1), Benzyltriethylammoniumchlorid (TEBA), 1,4,7,10,13,16-Hexaoxacyclooctadecan (18-Crown-6), Tetrabutylammoniumbromid, Tetrabutylammoniumhydrogensulfat, Tetrabutylammoniumjodid, Methyltrialkyl ($C_8$-$C_{10}$)-ammoniumchlorid (Adogen 464).

Die Verbindungen der Formeln (IV) und (V) werden in etwa äquimolarem Verhältnis zueinander eingesetzt. Ein Überschuß der einen oder anderen Komponente bringt keinen wesentlichen Vorteil.

Nach erfolgter Umsetzung wird das Verdünnungsmittel abdestilliert und die Verbindungen der Formel I in an sich bekannter Weise isoliert, indem sie z.B. aus dem Rückstand mit einem geeigneten Lösungsmittel z.B. Ether extrahiert werden. Die Verbindungen der Formel I können anschließend in üblicher Weise z.B. durch Destillation gereinigt werden.

Setzt man bei Verfahren 2c zur Herstellung der Verbindungen der Formel (I) als Verbindung der Formel (VI) 3-Chlorpropyloxy-pyridylphenylether und als Verbindung der Formel (VII) 4-Mercaptopyridin ein, läßt sich der Reaktionsverlauf durch folgendes Formelschema wiedergeben:

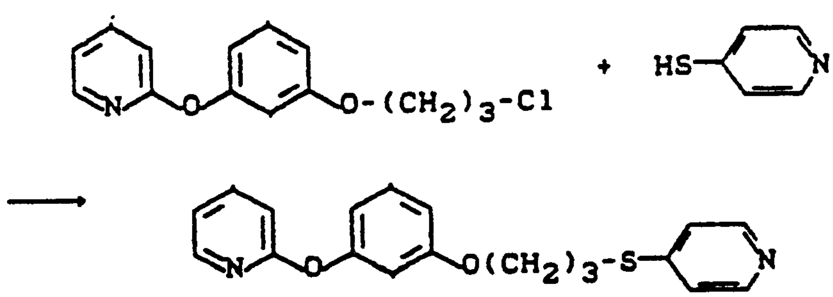

Das Verfahren wird bevorzugt angewendet, wenn die Verbindung der Formel (VII) eine Aminogruppe enthält.

Bevorzugt werden Verbindungen der Formeln (VI) und (VII) eingesetzt, in denen $R^1$, $R^2$, $R^3$, $R^4$, $R^5$, $R^6$, X, Y, Z, Het, m, n, o, p die bei den Verbindungen der Formel (I) angegebenen bevorzugten oder besonders bevorzugten Bedeutungen besitzen.

Im einzelnen seien folgende Verbindungen der Formel (VI) genannt:

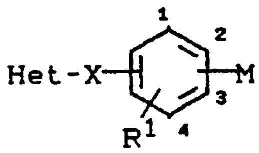

| Het | X | $R^1$ | M |
|---|---|---|---|
| 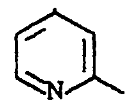 | $CH_2$ | 1-Cl | 3 - $O-CH_2-CH_2-Cl$ |
| 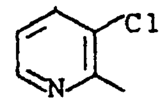 | O | H | 2-$O-CH_2CH_2-Cl$ |
| 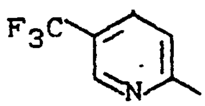 | S | H | 3-$O-CH_2CH_2-Cl$ |
| 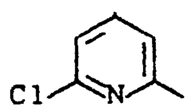 | O | H | 2-$O-CH_2CH_2CH_2-Cl$ |
| 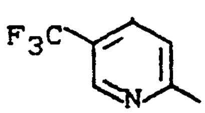 | O | H | 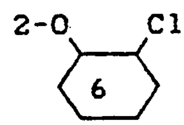 |
| 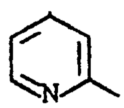 | O | H | 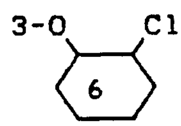 |

| Het | X | $R^1$ | M |
|---|---|---|---|
| 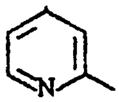 | O | H | 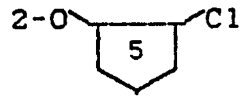 |
| 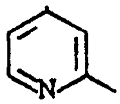 | $CH_2$ | H | 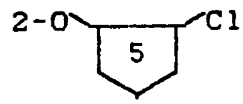 |
| 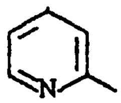 | O | H | $2-O-CH_2CH_2CH_2-Br$ |
| 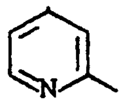 | O | H | $2-O-CH_2-\underset{\displaystyle CH_3}{\underset{\mid}{CH}}-Cl$ |
| 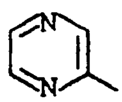 | O | H | $2-O-CH_2-\underset{\displaystyle CH_3}{\underset{\mid}{CH}}-Cl$ |
| 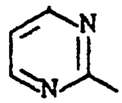 | O | H | $2-O-CH_2-\underset{\displaystyle C_2H_5}{\underset{\mid}{CH}}-Cl$ |

In einzelnen seien folgende Verbindungen der Formel VII genannt: 2-Hydroxypyridin, 2-Mercaptopyridin, 2-Hydroxypyrimidin, 3-Hydroxypyridin, 2-Amino-3-hydroxypyridin, 2-Chlor-3-hydroxypyridin, 2,3-Dihydroxypyridin.

Die Verbindungen der Formel (VI) und (VII) sind bekannt (z.B. EP-OS 3877, US-P 4 491 468).

Die Umsetzung erfolgt durch Erhitzen der Verbindungen (VI) und (VII) in Gegenwart von Verdünnungsmitteln, Basen sowie gegebenenfalls Katalysatoren.

Die Umsetzung erfolgt bei Temperaturen von 0 - 200° C, bevorzugt bei 20 - 100° C, besonders bevorzugt bei Siedetemperatur des Verdünnungsmittels.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Alkohole wie Methanol, Ethanol, Isopropanol, Butanol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Als Basen kommen anorganische und organische Basen in Frage. Als Basen seien genannt Alkali- und Erdalkalihydroxide, -carbonate, -hydrogencarbonate, -alkoholate, ferner Amine wie insbesondere tertiäre Amine z.B. Trimethylamin, Triethylamin, N-Methylmorpholin, Pyridin, Picoline, N-Ethylpyrrolidin, Diazabicyclo(4,3,0)-undecan(DBU), 1,4-Diazabicyclo(2,2,2)octan (DABCO), Diazabicyclo(3,2,0)nonen (DBN).

Als Katalysatoren kommen z.B. in Frage Phasentransferkatalysatoren wie Methyltrialkyl ($C_8$-$C_{10}$)-ammoniumchlorid (Adogen 464), 1,4,7,10,13,16-Hexaoxacyclooctadecan (18-Crown-6), Tetrabutylammoniumbromid, Tris-[2-(2-methoxyaethoxy)-aethyl]-amin (TDA-1), Benzyltriethylammoniumchlorid (TEBA).

Die Verbindungen der Formeln (VI) und (VII) werden in etwa äquimolarem Verhältnis zueinander

eingesetzt. Ein Überschuß der einen oder anderen Komponente bringt keinen wesentlichen Vorteil.

Nach erfolgter Umsetzung wird das Verdünnungsmittel abdestilliert und die Verbindungen der Formel I in an sich bekannter Weise isoliert, indem sie z.B. aus dem Rückstand mit einem geeigneten Lösungsmittel z.B. Ether extrahiert werden. Die Verbindungen der Formel (I) können anschließend in üblicher Weise z.B. durch Destillation gereinigt werden.

Setzt man bei Verfahren 2d als Verbindung der Formel (VIII) 1-Methyl-1-(3-hydroxyphenoxy)ethanol und als heterocyclische Verbindung 2-Chlorpyridin ein, so läßt sich der Reaktionsverlauf durch folgendes Reaktionsschema darstellen:

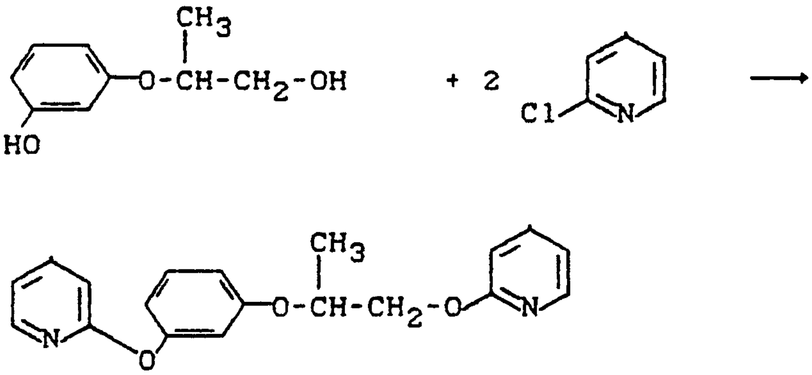

Bevorzugt werden Verbindungen der Formeln (VIII) und (IX) eingesetzt, in denen $R^1$-$R^6$, Het, X, Y, Z, m, n, o, p die bei den Verbindungen der Formel (I) angegebenen bevorzugten und besonders bevorzugten Bedeutungen besitzen.

Die Verbindungen der Formeln (VIII) und (IX) sind bekannt oder lassen sich analog in bekannten Verfahren herstellen.

Die Umsetzung erfolgt wie bei Verfahren 2b beschrieben.

Die Wirkstoffe eignen sich bei günstiger Warmblütertoxizität zur Bekämpfung von tierischen Schädlingen wie Arthropoden, vorzugsweise Insekten und Spinnentieren, die in der Tierhaltung und Tierzucht bei Haus- und Nutztieren sowie Zoo-, Labor-, Versuchs- und Hobbytieren vorkommen. Sie sind dabei gegen alle oder einzelne Entwicklungsstadien der Schädlinge sowie gegen resistente und normal sensible Arten der Schädlinge wirksam.

Durch die Bekämpfung der tierischen Schädlinge sollen Krankheiten und deren Übertragung, Todesfälle und Leistungsminderungen (z.B. bei der Produktion von Fleisch, Milch, Wolle, Häuten, Eiern) verhindert werden, so daß durch den Einsatz der Wirkstoffe eine wirtschaftlichere und einfachere Tierhaltung möglich ist bzw. in bestimmten Gebieten erst möglich wird.

Zu den Schädlingen gehören:

Aus der Ordnung der Anoplura z.B. Haematopinus spp., Linognathus spp., Solenopotes spp., Pediculus spp., Pthirus spp.;

aus der Ordnung der Mallophaga z.B. Trimenopon spp., Menopon spp., Eomenacanthus spp., Menacanthus spp., Trichodectes spp., Felicola spp., Damalinea spp., Bovicola spp;

aus der Ordnung der Diptera z.B. Chrysops spp., Tabanus spp., Musca spp., Hydrotaea spp., Muscina spp., Haematobosca spp., Haematobia spp., Stomoxys spp., Fannia spp., Glossina spp., Lucilia spp., Calliphora spp., Auchmeromyia spp., Cordylobia spp., Cochliomyia spp., Chrysomyia spp., Sarcophaga spp., Wohlfartia spp., Gasterophilus spp., Oesteromyia spp., Oedemagena spp., Hypoderma spp., Oestrus spp., Rhinoestrus spp., Melophagus spp., Hippobosca spp..

Aus der Ordnung der Siphonaptera z.B. Ctenocephalides spp., Echidnophaga spp., Ceratophyllus spp..

Aus der Ordnung der Metastigmata z.B. Hyalomma spp., Rhipicephalus spp., Boophilus spp., Amblyomma spp., Haemophysalis spp., Dermacentor spp., Ixodes spp., Argas spp., Ornithodorus spp., Otobius spp.;

aus der Ordnung der Mesastigmata z.B. Dermanyssus spp., Ornithonyssus spp., Pneumonyssus spp..

Aus der Ordnung der Prostigmata z.B. Cheyletiella spp., Psorergates spp., Myobia spp., Demodex spp., Neotrombicula spp.;

aus der Ordnung der Astigmata z.B. Acarus spp., Myocoptes spp., Psoroptes spp., Chorioptes spp., Otodectes spp., Sarcoptes spp., Notoedres spp., Knemidocoptes spp., Neoknemidocoptes spp. Lytodites spp., Laminosioptes spp..

Zu den Haus- und Nutztieren gehören Säugetiere wie z.B. Rinder, Schafe, Ziegen, Pferde, Schweine,

Hunde, Katzen, Kamele, Wasserbüffel, Esel, Kaninchen, Damwild, Rentiere, Pelztiere wie z.B. Nerze, Chinchilla, Waschbär, Vögel wie z.B. Hühner, Puten, Fasanen, Gänse, Enten.

Zu Labor- und Versuchstieren gehören z.B. Mäuse, Ratten, Meerschweinchen, Goldhamster, Hunde und Katzen.

Zu den Hobbytieren gehören z.B. Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Boli, Tränken, Granulaten, oral applizierbare Lösungen, Suspensionen oder Emulsionen, medikiertem Futter oder Trinkwasser. Die dermale Anwendung geschieht z.B. in Form des Tauchens (Dippen), Sprühens (Sprayen) oder Aufgießens (pour-on und spot-on) und des Einpuderns. Die parenterale Anwendung geschieht z.B. in Form der Injektion (z.B. intramusculär, subcutan, intravenös) oder durch Implantate.

Besonders hervorgehoben seien die Zubereitungen zur dermalen Anwendung. Dazu gehören Lösungen, Suspensions- und Emulsionskonzentrate sowie Mikroemulsionen, die vor Anwendung mit Wasser verdünnt werden, Aufgußformulierungen, Pulver und Stäube, Aerosole und wirkstoffhaltige Formkörper sowie dustbags, back-rubber.

Diese Zubereitungen werden in bekannter Weise hergestellt, z.B. durch Vermischen des Wirkstoffs mit Streckmitteln, also z.B. flüssigen Lösungsmitteln, gegebenenfalls unter Verwendung von oberflächenaktiven Mitteln, also Emulgiermitteln und/oder Dispergiermitteln. Im Falle der Benutzung von Wasser als Streckmittel können z.B. auch organische Lösungsmittel als Hilfslösungsmittel verwendet werden.

Zu den flüssigen Verdünnungsmitteln zählen neben Wasser Alkohole wie Methanol, Ethanol, Isopropanol, n-Butanol, Amylalkohol, Octanol;
Glykole wie Propylenglykol, 1,3-Butylenglykol, Ethylglykol, Dipropylenglykolmonomethylether;
Glycerin;
aromatische Alkohole wie Benzylalkohol;
Carbonsäureester wie z.B. Ethylacetat, Benzylbenzoat, Butylacetat, Propylencarbonat, Milchsäureethylester;
aliphatische Kohlenwasserstoffe wie Paraffine, Cyclohexan, Methylenchlorid, Ethylenchlorid;
aromatische Kohlenwasserstoffe wie Xylol, Toluol, Alkylnaphthaline, Chlorbenzole;
Ketone wie z.B. Aceton und Methylethylketon, Methylisobutylketon, Cyclohexanon;
natürliche und synthetische Mono- und Triglyceride mit natürlichen Fettsäuren wie Baumwollsaatöl, Erdnußöl, Maiskeimöl, Olivenöl, Ricinusöl, Sesamöl;
weiterhin Dimethylsulfoxid, Dimethylacetamid, Dimethylformamid, N-Methylpyrrolidon, Dioxan, 2,2-Dimethyl-4-oxymethyl-1,3-dioxolan.

Zu den oberflächenaktiven Stoffen zählen;
Emulgatoren und Netzmittel wie anionaktive Tenside, z.B. Alkylsulfonate, Alkylsulfate, Arylsulfonate, Na-Laurylsulfat, Fettalkoholethersulfate, Mono/Dialkylpolyglykoletherorthophosphorsäureester-Monoethanolaminsalz, Calciumalkylarylsulfonat;
kationaktive Tenside, z.B. Cetyltrimethylammoniumchlorid;
ampholytische Tenside, z.B. Di-Na-N-lauryl-beta-iminodipropionat oder Lecithin;
nichtionogene Tenside, z.B. polyoxyethyliertes Ricinusöl, polyoxyethyliertes Sorbitan-Monooleat, polyoxyethyliertes Sorbitan-Monostearat, Glycerinmonostearat, Polyoxyethylenstearat, Alkylphenolpolyglykolether, polyoxyethyliertes Sorbitanmonopalmitat, Polyoxyethylen-laurylether, Polyoxyethylen-oleylether, Polyoxyethylen-mannitanmonolaurat, Alkylpolyglykolether, Oleylpolyglykolether, Dodecylpolyglykolether, ethoxyliertes Nonylphenol, Isooctylphenylpolyethoxyethanol.

Die Zubereitungen können außerdem enthalten:
Haftvermittler, z.B. Carboxymethylcellulose, Methylcellulose und andere Cellulose- und Stärke-Derivate, Polyacrylate, Alginate, Gelatine, Gummi arabicum, Polyvinylpyrrolidon, Polyvinylalkohol, Copolymere aus Methylvinylether und Maleinsäureanhydrid, Polyethylenglykole, Paraffine, Öle, Wachse, hydriertes Rizinusöl, Lecithine und synthetische Phospholipide.

Die Zubereitungen können Farbstoffe wie anorganische Pigmente, z.B.Eisenoxid, Titanoxid, Ferrocyanblau und organische Farbstoffe, wie Alizarin-, Azo- und Metallphthalocyaninfarbstoffe enthalten.

Die Zubereitungen können Spreitmittel enthalten, z.B. Silikonöle verschiedener Viskosität, Fettsäureester wie Ethylstearat, Di-n-butyl-adipat, Laurinsäurehexylester, Dipropylen-glykolpelargonat, Ester einer verzweigten Fettsäure mittlerer Kettenlänge mit gesättigten Fettalkoholen $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester,

Dibutylphthalat, Adipinsäurediisopropylester, letzterem verwandte Estergemische u.a.;
Triglyceride wie Capryl/Caprinsäuretriglycerid, Triglyceridgemische mit Pflanzenfettsäuren der Kettenlänge $C_8$-$C_{12}$ oder anderen speziell ausgewählten natürlichen Fettsäuren, Partialglyceridgemische gesättigter oder ungesättigter evtl. auch hydroxylgruppenhaltige Fettsäuren, Monodiglyceride der $C_8$/$C_{10}$-Fettsäuren und andere;
Fettalkohole wie Isotridecylalkohol, 2-Octyldodecanol, Cetylstearylalkohol, Oleylalkohol.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind gegebenenfalls gebrochene und fraktionierte, z.B. synthetische und natürliche Gesteinsmehle wie Kaoline, Talkum, Kreide, Quarz, Diatomeenerde, Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs-und Futtermittel wie Milchpulver, Tiermehle, Getreidemehle und -schrote, Stärken, Sägemehl.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in Form ihrer oben erwähnten festen oder flüssigen Formulierungen auch eingekapselt vorliegen.

Die Wirkstoffe können auch in Form eines Aerosols angewendet werden. Dazu wird der Wirkstoff in geeigneter Formulierung unter Druck fein verteilt.

Es kann auch vorteilhaft sein, die Wirkstoffe in Formulierungen anzuwenden, die den Wirkstoff verzögert frei geben. Als solche seien wirkstoffhaltige Formkörper wie z.B. Platten, Bänder, Streifen, Halsbänder, Ohrmarken, Schwanzmarken, Gliedmaßenbänder, Halfter, Markierungsvorrichtungen genannt. Als solche seien auch wirkstoffhaltige Implantate und Boli genannt.

Die Verabreichung der Wirkstoffe kann auch zusammen mit dem Futter und/oder dem Trinkwasser erfolgen.

Die Wirkstoffe können in den Formulierungen allein oder in Mischung mit anderen Wirkstoffen oder Synergisten vorliegen.

Direkt angewendete Formulierungen enthalten zwischen $10^{-7}$ und 5 Gewichtsprozent, bevorzugt zwischen $10^{-4}$ und 1 Gewichtsprozent Wirkstoff.

Formulierungen die erst nach weiterer Verdünnung angewendet werden enthalten 1 - 95 Gewichtsprozent, bevorzugt 5 - 90 Gewichtsprozent Wirkstoff.

## Formulierungsbeispiele

1. Herstellung eines Emulsionskonzentrates

| | |
|---|---|
| a) Wirkstoff gemäß Beispiel 1 (100 %) | 25,00 g |
| nichtionischer Emulgator (Emulgator 368® = Alkylarylpolyglycolether MG ca. 1165) | 25,00 g |
| Dipropylenglykolmonomethylether ad | 100,00 ml |

### Herstellung

Die Substanzen werden zusammen gewogen und so lange gerührt, bis eine klare Lösung entstanden ist.

Vor Gebrauch wird die Lösung auf ihre Anwendungskonzentration verdünnt.

| | |
|---|---|
| b) Wirkstoff gemäß Beispiel 2 (100 %) | 1,00 g |
| Polyoxyethylenstearat | 0,50 g |
| Sorbitan-Sesquioleat | 0,40 g |
| Wasser | 4,00 g |
| Polyethylenglykol 200 ad 100 ml | |

Herstellung und Anwendung wie bei 1a

2. Herstellung einer pour-on-Formulierung

| a) Zusammensetzung | |
|---|---|
| Wirkstoff gemäß Beispiel 1 (100 %) | 5,00 g |
| Isopropylmyristat | 30,00 g |
| 2-Octyldodecanol | 20,00 g |
| Isopropanol ad 100 ml | |

Herstellung:

Die Substanzen werden zusammen gewogen und bis zur klaren Lösung gerührt.

| b) Zusammensetzung | |
|---|---|
| Wirkstoff gemäß Beispiel 1 (100 %) | 0,50 g |
| Silikonöl 100 | 30,00 g |
| Butylacetat ad 100 ml | |

Herstellung wie bei Beispiel a)

| 3. Herstellung einer Mikroemulsion | |
|---|---|
| Wirkstoff gemäß Beispiel 1 (100 %) | 13,00 g |
| Eumulgin B3®(Alkylarylpolyglycolether) | 30,00 g |
| Cetiol HE® (Polyolfettsäureester) | 30,00 g |
| Isopropylmyristat | 5,00 g |
| Benzylalkohol | 3,00 g |
| Wasser ad 100 ml | |

Herstellung:

Der Wirkstoff wird in den lipophilen Komponenten (Eumulgin, Cetiol, Benzylalkohol, Isopropylmyristat) dispergiert.

Nach Erwärmen auf 60 °C wird Wasser der gleichen Temperatur zugemischt und abgekühlt. Die entstandene Mikroemulsion gleicht außerlich einer klaren Lösung.

4. Herstellung einer Sprühformulierung

| Zusammensetzung | |
|---|---|
| Wirkstoff gemäß Beispiel 1 (100 %) | 20 g |
| Emulgator Toximul® (Mischung aus Alkylbenzolsulfonat-Ca und nichtionogenen Emulgatoren und Methanol) | 7 g |
| Emulgator Toximul S® (Mischung aus Alkylbenzolsulfonat-Ca und nichtionogenem Emulgator und Methanol) | 5 g |
| Sovesso 200® (Alkylnaphthalin-Gemisch hochsiedender Erdölfraktionen) | ad 100 ml |

Herstellung:

Der Wirkstoff wird mit den restlichen Komponenten zusammen gewogen, gerührt und vor Anwendung mit Wasser auf die Anwendungskonzentration verdünnt.

Anwendungsbeispiele

A. In vitro-Test an Flöhen (alle Entwicklungsstadien)

Testobjekt: Alle Stadien (Eier, Larven, Puppen, Adulte) von Ctenocephalides felis

Testverfahren

Die zu testende Substanz wird in der gewünschten Konzentration homogen in Blutmehl verteilt. In dieses Gemisch aus Blutmehl und Testsubstanz werden Floheier, die von flohbefallenen Katzen gesammelt wurden, gebracht und bei 80 % rel. Feuchte und 25° C inkubiert.

Nach Ablauf der Entwicklungszeit von 3 bis 4 Wochen wird festgestellt, ob adulte Flöhe zur Entwicklung gekommen sind. Dabei bedeutet eine 100 %ige Wirkung, daß keine adulten Flöhe festgestellt wurden und 0 %, daß lebende adulte Flöhe festgestellt wurden.

Der Wirkstoff gemäß Beispiel 1 zeigt bei 5 ppm 100 % Wirkung.

B. In vivo-Test an Flöhen (alle Entwicklungsstadien)

Testobjekt: Eier, Larven, Puppen von Ctenocephalides felis in der Umgebung von Hunden/Katzen, adulte Flöhe auf Hunden/Katzen.

Testverfahren

Läger von flohfreien Hunden/Katzen in Boxen werden mit der zu testenden Substanz in der gewünschten Anwendungskonzentration besprüht. Zu bestimmten Zeiten nach der Applikation werden Eier von Ctenocephalides felis, die von behandelten Katzen gesammelt wurden, auf die Läger der Hunde/Katzen gebracht. Es wird festgestellt, ob und zu welchem Zeitpunkt die in den Boxen befindlichen Hunde/Katzen von adulten Flöhen befallen werden.

Dabei bedeutet eine 100 %ige Wirkung, daß Hunde/Katzen keinen Flohbefall aufweisen und 0 %

Wirkung, daß Hunde/Katzen von Flöhen befallen sind.

Der Wirkstoff gemäß Beispiel 1 zeigt 100 % Wirkung.

## Herstellbeispiele für Verfahren 2a

Allgemeine Vorschrift:

Ein Gemisch aus 1 Mol der Verbindung der Formel (II) und der etwa 1,1- bis 2-fachen molaren Menge Verbindung der Formel (III) und der etwa 1- bis 4-fachen molaren Menge $K_2CO_3$-Pulver werden in Acetonitril etwa 24 Stunden am Rückfluß gekocht. Nach dem Abkühlen wird von den Salzen abfiltriert und das Filtrat im Vakuum eingeengt. Das entstandene Produkt wird durch Kugelrohrdestillation, Umkristallisation oder Säulenchromatographie weiter gereinigt.

Gemäß dieser Vorschrift werden die folgenden Verbindungen erhalten.

| Bsp. Nr. | Het | X | 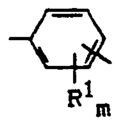 | $[O\,(C)_n-(C-C)_o]_p$-Y- | Z | Siedep. (°C/Druck) oder Schmp. °C |
|---|---|---|---|---|---|---|
| 1 | 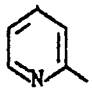 | O | 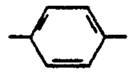 | $-O-CH_2CH_2-O-$ | 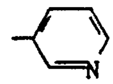 | 210 - 230°C / 0,4 mm |
| 2 | 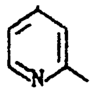 | O | 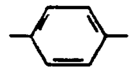 | $-O-CH_2CH_2CH_2-O-$ | 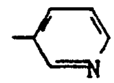 | 220 - 240°C / 0,4 mm |
| 3 | 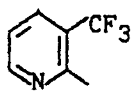 | O | 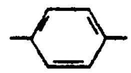 | $-O-CH_2CH_2CH_2-O-$ | 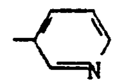 | 240 - 250°C / 0,3 mm |
| 4 | 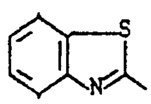 | O | 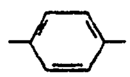 | $-O-CH_2CH_2-O-$ | 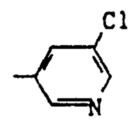 | 230 - 240°C / 0,1 mm |
| 5 | 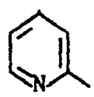 | O | 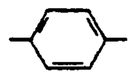 | $-O-CH_2CH_2-O-$ | 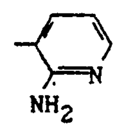 | 230 - 240°C / 0,1 mm |
| 6 | 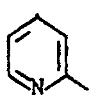 | O | 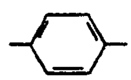 | $-O-CH_2CH_2-O-$ | 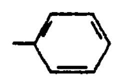 | 98°C |
| 7 | 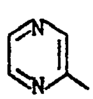 | O | 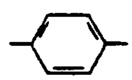 | $-O-CH_2CH_2-O-$ | 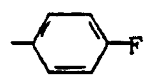 | 220 - 240°C / 0,3 mm |
| 8 | 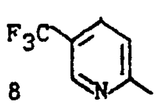 | O | 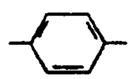 | $-O-CH_2CH_2-O-$ | 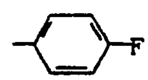 | 65°C |

| Bsp. Nr. | Het | X | 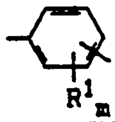 | $[O-(C)_n-(C-C-O)]_p-Y-$ | Z | Siedep. (°C/Druck) oder Schmp. °C |
|---|---|---|---|---|---|---|
| 9 | 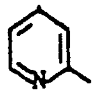 | O | 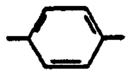 | $-OCH_2CH_2-O-$ | 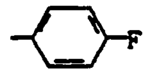 | 92°C |
| 10 | 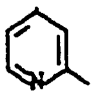 | O | 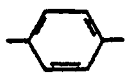 | $-O-CH_2CH_2-O-$ | 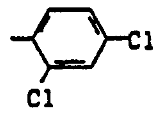 | 128°C |
| 11 | 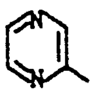 | O | 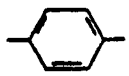 | $-OCH_2CH_2-O-$ | 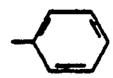 | 210 - 235°C / 0,3 mm |
| 12 | 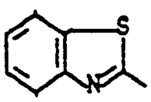 | O | 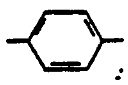 | $-OCH_2CH_2-O-$ | 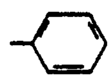 | 240°C / 0,3 mm |
| 13 | 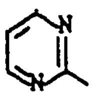 | O | 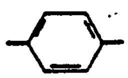 | $-OCH_2CH_2-O-$ | 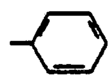 | 105°C |
| 14 | 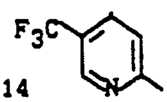 | O | 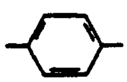 | $-OCH_2CH_2-S-$ | 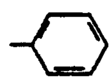 | 230°C / 0,3 mm |
| 15 | 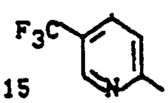 | O | 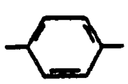 | $-OCH_2CH_2-O-$ | 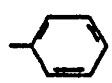 | 210 - 225°C 0,3 mm |
| 16 | 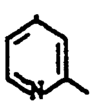 | O | 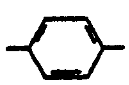 | $-OCH_2CH_2-O-$ | 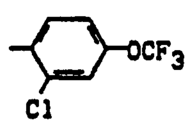 | 125-128°C |
| 17 | 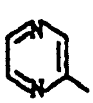 | O | 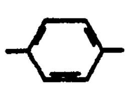 | $-OCH_2CH_2-O-$ | 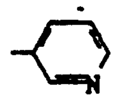 | 113-115°C |

| Bsp. Nr. | Het | X | 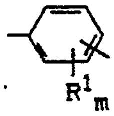 | $-[O(C)_n-(C-C)_o]_p-Y-$ | Z | Siedep. (°C/Druck) oder Schmp. °C |
|---|---|---|---|---|---|---|
| 18 | 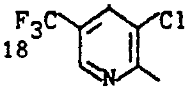 | O | 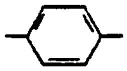 | $-OCH_2CH_2-O-$ | 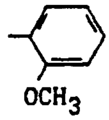 | 121-123°C |
| 19 | 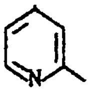 | O | 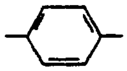 | $-O-CH_2CH_2-O-$ | 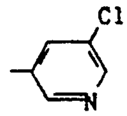 | 53°C |
| 20 | 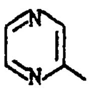 | O | 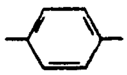 | $-OCH_2CH_2-O-$ | 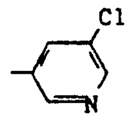 | 93°C |
| 21 | 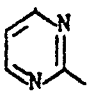 | O | 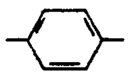 | $-OCH_2CH_2-O-$ | 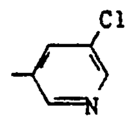 | 104°C |
| 22 | 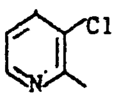 | O | 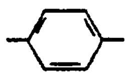 | $-OCH_2CH_2CH_2-O-$ | 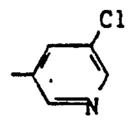 | 240°C / 0,1 mm |
| 23 | 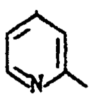 | O | 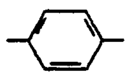 | $-OCH_2CH_2CH_2-O-$ | 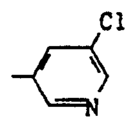 | 58°C |
| 24 | 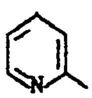 | O | 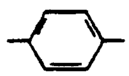 | $-OCH_2CH_2CH_2-O-$ | 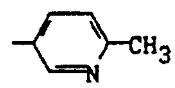 | 230°C / 0,3 mm |
| 25 | 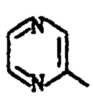 | O | 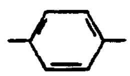 | $-OCH_2CH_2-S-$ | 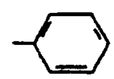 | 220°C / 0,4 mm |

| Bsp. Nr. | Het | X | 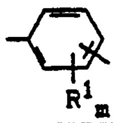 | $[O-(C)_n-(C-C-O)]_p$-Y- | Z | Siedep. (°C/Druck) oder Schmp. °C |
|---|---|---|---|---|---|---|
| 26 | 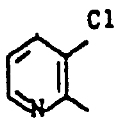 | O | 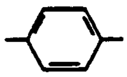 | $-OCH_2CH_2-S-$ | 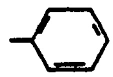 | 240° C / 0,2 mm |
| 27 | 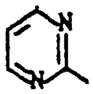 | O | 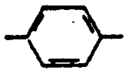 | $-OCH_2-O-$ | 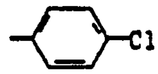 | 230° C / 0,3 mm |
| 28 | 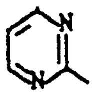 | O | 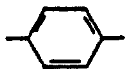 | $-OCH_2CH_2-S-$ | 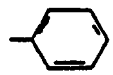 | 230° C / 0,4 mm |
| 29 | 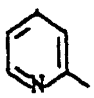 | O | 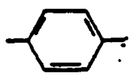 | $-OCH_2-O-$ | 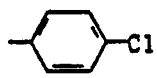 | 210° C / 0,4 mm |
| 30 | 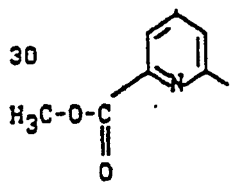 | O | 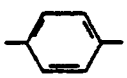 | $-OCH_2CH_2-O-$ | 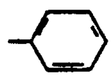 | 240° C / 0,2 mm |
| 31 | 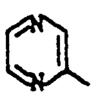 | O | 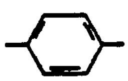 | $-OCH_2-O-$ | 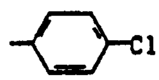 | 220-240° C / 0,4 mm |
| 32 | 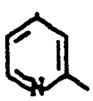 | O | 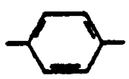 | $-O-CH_2CF_2-O-$ | 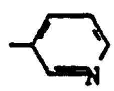 | 240° C / 0,2 mm |
| 33 | 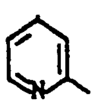 | O | 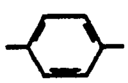 | $-O-CH_2CF_2-O-$ | 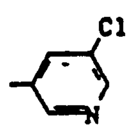 | 240-250° C / 0,1 mm |

| Bsp. Nr. | Het | X | 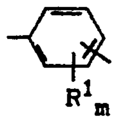 $[O\ (C)_n-(C-C)_o]_p$-Y- | | Z | Siedep. (°C/Druck oder Schmp. °C |
|---|---|---|---|---|---|---|
| 33a | 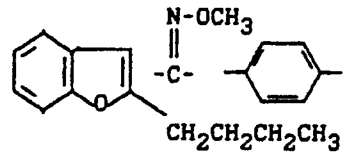 | | | -O-$CH_2CH_2$-O- | 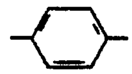 | 250° C/ 0,1 mm |
| 33b | 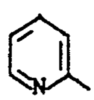 | O | 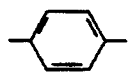 | -O-$CH_2CH_2$-O- | 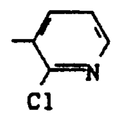 | 91 - 92° C |
| 33c | 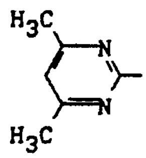 | S | 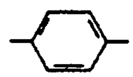 | -O-$CH_2CH_2CH_2$-O- | 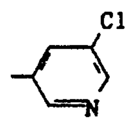 | 240° C/ 0,1 mm |
| 33d | 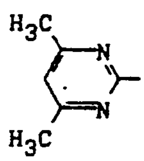 | S | 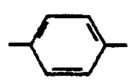 | -O-$CH_2CH_2$-O- | 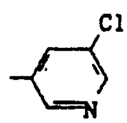 | 230° C/ 0,1 mm |

Herstellbeispiele für Verfahren 2b

Allgemeine Vorschrift:

Ein Gemisch aus 1 Mol der Verbindung der Formel (IV) und der etwa 1,1- bis 3-fachen molaren Menge Verbindung der Formel (V), der etwa 4-fachen molaren Menge NaOH-Pulver, der etwa 1,1-fachen molaren Menge $K_2CO_3$-Pulver sowie der etwa 0,02-fachen Menge des Phasentransferkatalysators Tris-(2-(2-methoxyethoxy)-ethyl)-amin werden in Toluol etwa 36 Stunden am Rückfluß gekocht. Nach dem Abkühlen wird die Toluolphase mit Wasser gewaschen, über $Na_2SO_4$ getrocknet und im Vakuum eingeengt. Das entstandene Produkt wird durch Kugelrohrdestillation, Umkristallisation oder Säulenchromatographie weiter gereinigt.

Gemäß dieser Vorschrift werden die folgenden Verbindungen erhalten.

| Bsp. Nr. | Het | X | 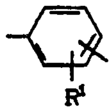 | $\left[O\ (C)_n-(C-C)_o\right]_p-Y-$ | Z | Siedep. (°C/Druck oder Schmp. °C |
|---|---|---|---|---|---|---|
| 34 | 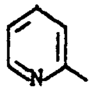 | O | 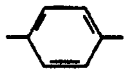 | $-O-CH_2-CH-O-$<br>$CH_3$ | 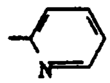 | 210 - 230°C / 0,4 mm<br>56°C |
| 35 | 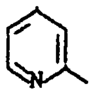 | O | 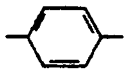 | $-O-CH-CH_2-O-$<br>$CH_3$ | 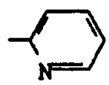 | 215 - 235°C / 0,4 mm<br>Schmp.: 76°C |
| 36 | 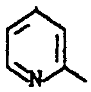 | O | 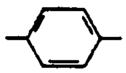 | $-O-CH_2CH_2-O-$ | 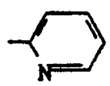 | 205 - 225°C / 0,4 mm |
| 37 | 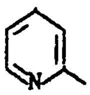 | O | 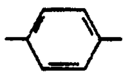 | $-O-CH_2-CH-O-$<br>$CF_3$ | 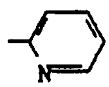 | 220 - 240°C / 0,4 mm |
| 38 | 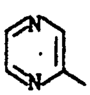 | O | 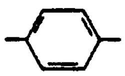 | $-O-CH_2-CH-O-$<br>$CH_3$ | 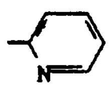 | 230°C / 0,4 mm |
| 39 | 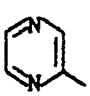 | O | 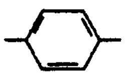 | $-O-CH-CH_2-O-$<br>$CH_3$ | 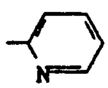 | 230°C / 0,4 mm |
| 40 | 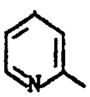 | $-CH_2-$ | 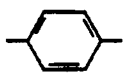 | $-O-CH_2CH-O-$<br>$CH_3$ | 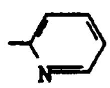 | 235°C / 0,4 mm |
| 41 | 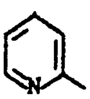 | $-CH_2-$ | 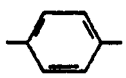 | $-O-CH-CH_2-O-$<br>$CH_3$ | 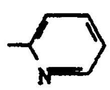 | 230°C / 0,4 mm |

| Bsp. Nr. | Het | X | 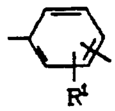 | $[O\ (C)_n-(C-C)_o]_p-Y-$ | Z | Siedep. (°C/Druck) oder Schmp. °C |
|---|---|---|---|---|---|---|
| 42 | 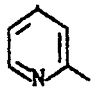 | -O- | 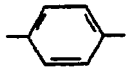 | $-O-CH_2CH-O-$<br>$CH_3$ | 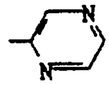 | 230°C / 0,4 mm |
| 43 | 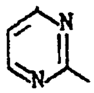 | -O- | 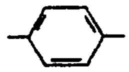 | $-O-CH_2-CH-O-$<br>$CH_3$ | 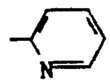 | 235°C / 0,4 mm |
| 44 | 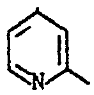 | -O- | 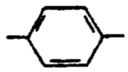 | $-O-CH_2CH-O-$<br>$CH_3$ | 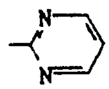 | gelbes Wachs |
| 45 | 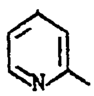 | -O- | 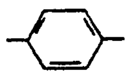 | $-O-CH_2CH-O-CH_2-$<br>$CH_3$ | 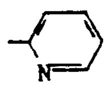 | 240 - 250°C / 0,4 mm |
| 46 | 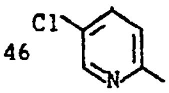 | O | 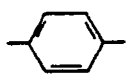 | $-O-CH_2CH_2-O-CH_2CH_2-O-$ | 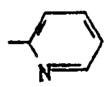 | 220 - 230°C / 0,2 mm |
| 47 | 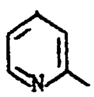 | O | 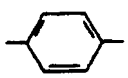 | 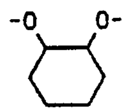 | 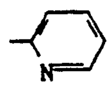 | 230°C / 0,3 mm |

| Bsp. Nr. | Het | X | 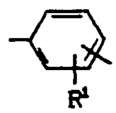 | $[O\ (C)_n-(C-C)_o]_p-Y-$ | Z | Siedep. (°C/Druck) oder Schmp. °C |
|---|---|---|---|---|---|---|
| 48 | 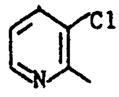 | -O- | 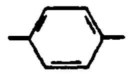 | $-O-CH_2CH-O-$<br>$CH_3$ | 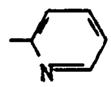 | 230° C / 0,3 mm |
| 49 | 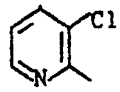 | -O- | 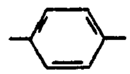 | $-O-CH_2-CH-O-$<br>$CH_3$ | 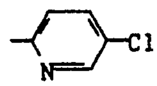 | 240° C / 0,2 mm |
| 50 | 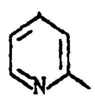 | -O- | 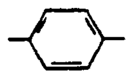 | $-O-CH_2CH-O-$<br>$CH_3$ | 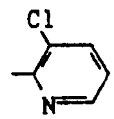 | 225° C 0,4 mm |
| 51 | 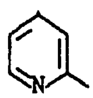 | -O- | 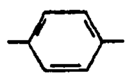 | $-O-CH_2CH-O-$<br>$CH_3$ | 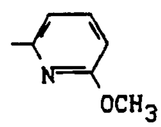 | 240 / 0,3 mm |
| 52 | 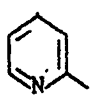 | O | 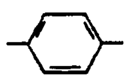 | $-O-CH_2CH-O-$<br>$CH_3$ | 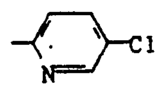 | 230° C / 0,4 mm |
| 53 | 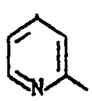 | O | 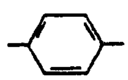 | $-O-CH_2CH-O-$<br>$CH_3$ | 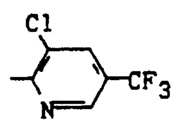 | 230-250° C / 0,3 mm |
| 54 | 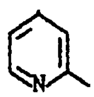 | O | 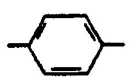 | $-O-CH_2CH-O-$<br>$CH_3$ | 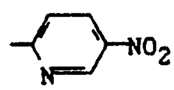 | 230° C / 0,1 mm |
| 55 | 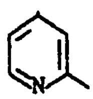 | O | 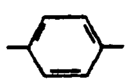 | $-O-CH_2CH-O-$<br>$CH_3$ | 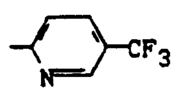 | 240 / 0,4 mm |

| Bsp. Nr. | Het | X | 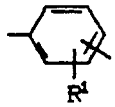 | -[O (C)$_n$-(C-C)$_O$]$_p$-Y- | Z | Siedep. (°C/Druck) oder Schmp. °C |
|---|---|---|---|---|---|---|
| 56 | 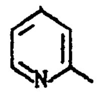 | O | 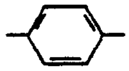 | 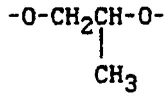 | 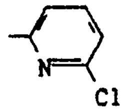 | 230° C / 0,4 mm |
| 57 | 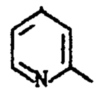 | O | 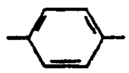 | 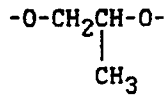 | 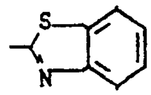 | 240° C / 0,2 mm |
| 58 | 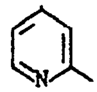 | O | 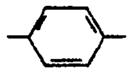 | 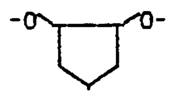 | 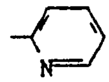 | 230° C / 0,3 mm |
| 59 | 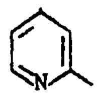 | O | 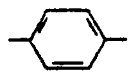 | 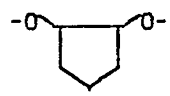 | 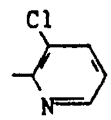 | 230-250° C / 0,2 mm |
| 60 | 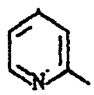 | O | 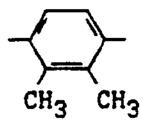 | 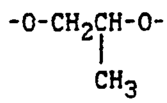 | 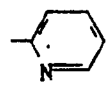 | 240° C / 0,2 mm |
| 61 | 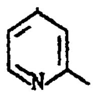 | O | 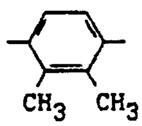 | 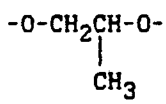 | 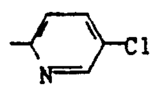 | 240-250° C / 0,2 mm |
| 62 | 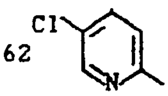 | O | 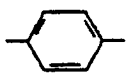 | 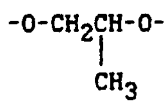 | 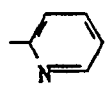 | 230 / 0,3 mm |
| 63 | 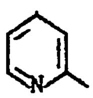 | O | 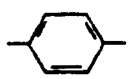 | 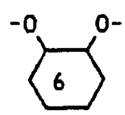 | 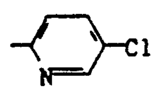 | 240° C / 0,2 mm |

| Bsp. Nr. | Het | X | 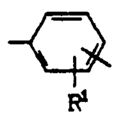 | $\left[O\ (C)_n-(C-C)_o\right]_p-Y-$ | Z | Siedep. (°C/Druck) oder Schmp. °C |
|---|---|---|---|---|---|---|
| 64 | 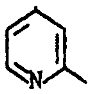 | O | 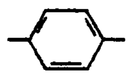 | $-O-CH_2CH-O-$<br>$CH_2CH_3$ | 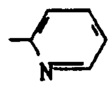 | 220° C/<br>0,3 mm |
| 65 | 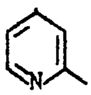 | O | 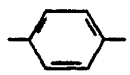 | $-O-CH_2CH-O-$<br>$CH_2CH_3$ | 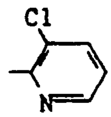 | 230° C/<br>0,3 mm |
| 66 | 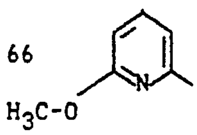 | O | 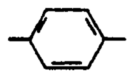 | $-O-CH_2CH-O-$<br>$CH_3$ | 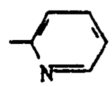 | 220-240° C /<br>0,3 mm |
| 67 | 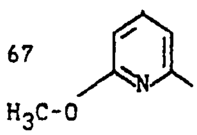 | O | 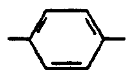 | $-O-CH_2CH-O-$<br>$CH_3$ | 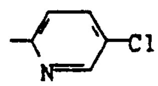 | 230-250° C<br>0,25 mm |
| 68 | 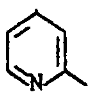 | O | 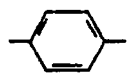 | $-O-CH-CH-O-$<br>$CH_3\ CH_3$ | 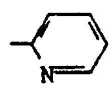 | 220-240° C /<br>0,3 mm |
| 69 | 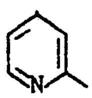 | O | 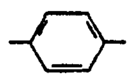 | $-O-CH-CH-O-$<br>$CH_3\ CH_3$ | 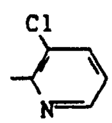 | 250° C/<br>0,3 mm |
| 70 | 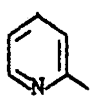 | O | 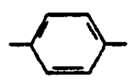 | $-O-CH_2CH-O-$<br>$CH_2-O-CH_2-CH=CH_2$ | 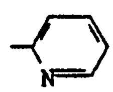 | 240° C /<br>0,2 mm |
| 71 | 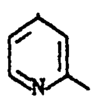 | O | 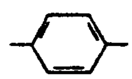 | $-O-CH_2CH-O-$<br>$CH_3$ | 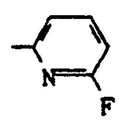 | 240° C /<br>0,3 mm |

| Bsp. Nr. | Het | X | 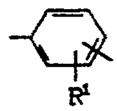 | $\left[O\,(C)_n-(C-C)_o\right]_p-Y-$ | Z | Siedep. (°C/Druck) oder Schmp. °C |
|---|---|---|---|---|---|---|
| 72 | 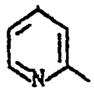 | O | 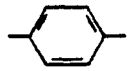 | $-O-CH_2CH_2-O-CH_2CH_2-O-CH_2CH_2-O-CH_2CH_2O-$ | 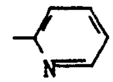 | 240°C / 0,1 mm |
| 73 | 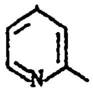 | O | 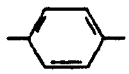 | $-O-CH_2CH_2-O-CH_2CH_2-O-$ | 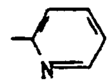 | 240°C / 0,2 mm |
| 74 | 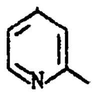 | O | 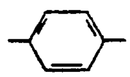 | $-O-CH_2CH(CH_2-O-CH_3)-O-$ | 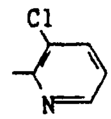 | 38 - 40°C |
| 75 | 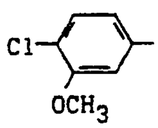 | O | 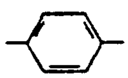 | $-O-CH_2CH(CH_3)-O-CH_2CH(CH_3)-O-$ | 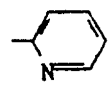 | 250°C / 0,2 mm |
| 76 | 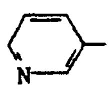 | O | 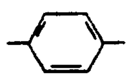 | $-O-CH_2CH(CH_3)-O-$ | 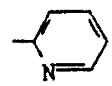 | 230°C / 0,2 mm |
| 77 | 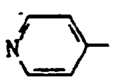 | $CH_2$ | 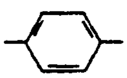 | $-O-CH_2CH(CH_3)-O-$ | 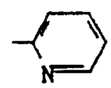 | 240°C / 0,3 mm |
| 78 | 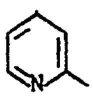 | O | 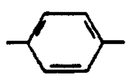 | $-O-CH_2CH(CH_2-O-CH_3)-O-$ | 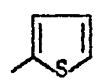 | 220°C / 0,25 mm |
| 79 | 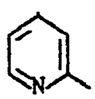 | $-CH_2O-$ | 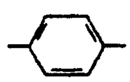 | $-O-CH_2CH(CH_2-O-CH_3)-O-$ | 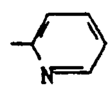 | 230°C / 0,3 mm |
| 80 | 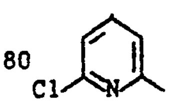 | O | 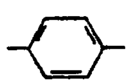 | $-O-CH_2CH(CH_2-O-CH_3)-O-$ | 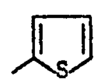 | 230-240°C / 0,2 mm |

| Bsp. Nr. | Het | X | 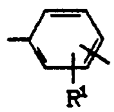 | $[O\ (C)_n-(C-C)_o]_p$ Y- | Z | Siedep. (°C/Druck) oder Schmp. °C |
|---|---|---|---|---|---|---|
| 81 | 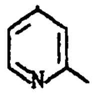 | O | 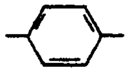 | -O-CH$_2$CH-O-<br>CH$_2$-O-CH$_3$ | 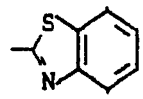 | 250° C / 0,1 mm |
| 82 | 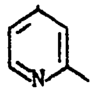 | O | 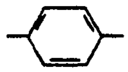 | -O-CH$_2$CH-O-<br>CH$_2$-O-CH$_2$-CH=CH$_2$ | 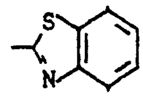 | 250° C / 0,1 mm |
| 83 | 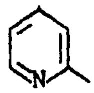 | O | 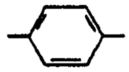 | -O-CH$_2$CH-O-<br>CH$_2$-O-CH$_2$-CH=CH$_2$ | 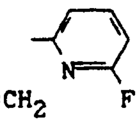 | 240° C / 0,2 mm |
| 84 | 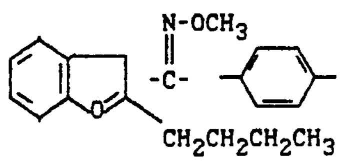 | | | -O-CH$_2$CH-O-<br>CH$_3$ | 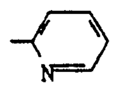 | 250° C / 0,1 mm |
| 85 | 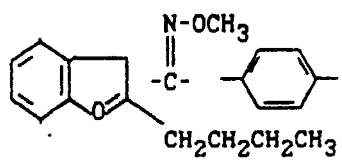 | | | -O-CH$_2$CH-O-<br>CH$_3$ | 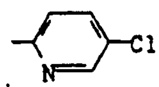 | 250° C / 0,1 mm |
| 86 | 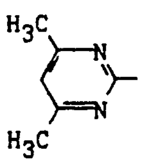 | S | 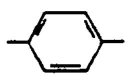 | -O-CH$_2$CH-O-<br>CH$_3$ | 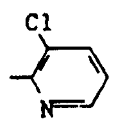 | 240° C/ 0,1 mm |
| 87 | 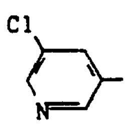 | O | 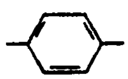 | -O-CH$_2$CH-O-<br>CH$_3$ | 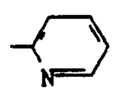 | 240° C/ 0,2 mm |
| 88 | 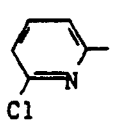 | O | 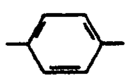 | -O-CH$_2$CH-O-<br>CH$_3$ | 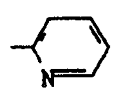 | 240° C/ 0,2 mm |

| Bsp. Nr. | Het | X | 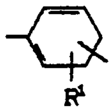 | $-[O\ (C)_n-(C-C)_O]_p-Y-$ | Z | Siedep. (°C/Druck) oder Schmp. °C |
|---|---|---|---|---|---|---|
| 89 | 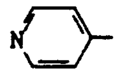 | O | 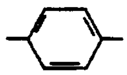 | -O-CH$_2$CH(CH$_3$)-O- | 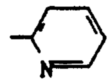 | 235° C/ 0,2 mm |
| 90 | 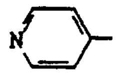 | O | 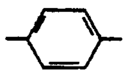 | -O-CH$_2$CH(CH$_3$)-O-CH$_2$CH(CH$_3$)-O-CH$_2$CH(CH$_3$)-O | 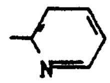 | 250° C/ 0,1 mm |
| 91 | 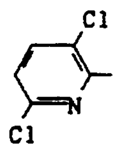 | O | 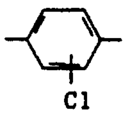 | -O-CH$_2$CH(CH$_3$)-O- | 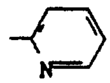 | 240° C/ 0,1 mm |
| 91a | 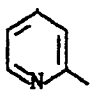 | O | 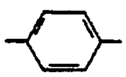 | -O-CH$_2$-CH(CH$_3$)-O- | 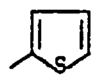 | 230° C/ 0,1 mm |
| 91b | 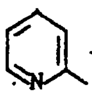 | -CH$_2$O- | 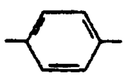 | -O-CH$_2$-CH(CH$_3$)-O- | 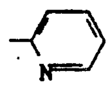 | 240° C/ 0,1 mm |
| 91c | 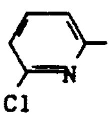 | O | 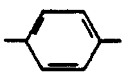 | -O-CH$_2$-CH(CH$_3$)-O- | 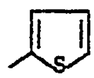 | 230° C/ 0,1 mm |
| 91d | 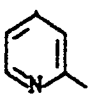 | S | 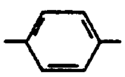 | -O-CH$_2$-CH(CH$_3$)-O- | 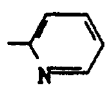 | 240° C/ 0,2 mm |

| Bsp. Nr. | Het | X | 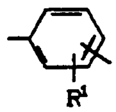 | 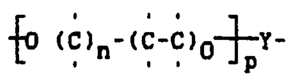 | Z | Siedep. (°C/Druck) oder Schmp. °C |
|---|---|---|---|---|---|---|
| 91e | 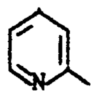 | O | 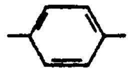 | 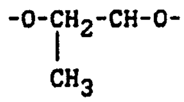 | 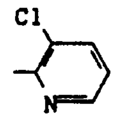 | 240° C/ 0,1 mm |
| 91f | 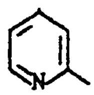 | O | 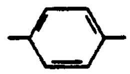 | 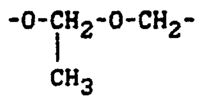 | 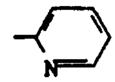 | 240° C/ 0,1 mm |
| 91g | 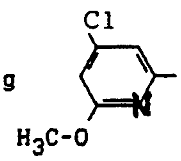 | O | 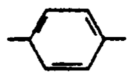 | 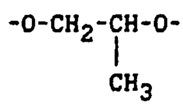 | 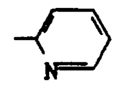 | 250° C/ 0,1 mm |

Herstellbeispiele für Verfahren 2c

Allgemeine Vorschrift:

Ein Gemisch aus 1 Mol der Verbindung der Formel (VI) und der etwa 0,8- bis 1,5-fachen molaren Menge Verbindung der Formel (VII), der etwa 2- bis 3-fachen molaren Menge $K_2CO_3$-Pulver sowie der etwa 0,02-fachen Menge des Phasentransferkatalysators TDA-1 werden in trockenem Acetonitril etwa 8 bis 36 Stunden bei 80 bis 160 °C gerührt. Nach dem Abkühlen wird das Acetonitril im Vakuum entfernt, der verbleibende Rückstand mit $H_2O$ versetzt und mit einem organischen Lösungsmittel extrahiert. Die vereinigten organischen Phasen werden nach dem Trocknen über $Na_2SO_4$ (z.B.) im Vakuum eingeengt. Das entstandene Produkt wird durch Kugelrohrdestillation, Umkristallisation oder Säulenchromatographie weiter gereinigt.

Gemäß dieser Vorschrift werden die folgenden Verbindungen erhalten.

| Bsp. Nr. | Het | X | 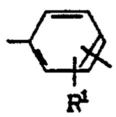 | $\left[O\,(C)_n-(C-C)_o\right]_p-Y-$ | Z | Siedep. (°C/Druck oder Schmp. °C |
|---|---|---|---|---|---|---|
| 92 | 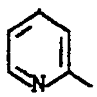 | O | 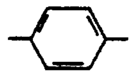 | $-O-CH_2CH_2-O-$ | 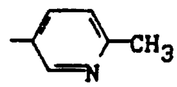 | 220 - 240°C / 0,3 mm |
| 93 | 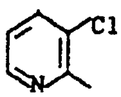 | O | 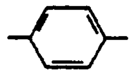 | $-OCH_2CH-CH_2-O-$<br>\|<br>$CH_3$ | 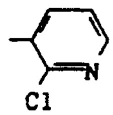 | 230 - 240°C / 0,2 mm |
| 94 | 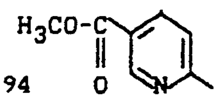 | O | 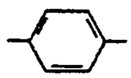 | $-OCH_2CH_2-O-$ | 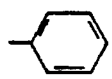 | 225 - 240°C / 0,3 mm |
| 95 | 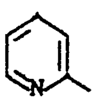 | $-CH_2-O-$ | 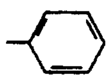 | $-O-CH_2CH_2-O-$ | 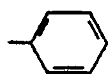 | 220°C / 0,4 mm |
| 96 | 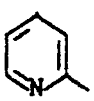 | -O- | 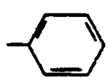 | $-O-CH_2-CH_2-S-$ | 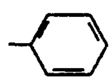 | 225 - 230°C / 0,4 mm |

Herstellbeispiele für Verfahren 2d

Allgemeine Vorschrift:

Ein Gemisch aus 1 Mol der Verbindung der Formel (VIII) und der etwa 3- bis 6-fachen molaren Menge Verbindung der Formel (IX), der etwa 2- bis 3-fachen molaren Menge $K_2CO_3$-Pulver, der etwa 4-fachen molaren Menge NaOH-Pulver sowie der etwa 0,02-fachen molaren Menge des Phasentransferkatalysators TDA-1 werden in Toluol ca. 36 Stunden am Rückfluß gekocht. Nach dem Abkühlen wird die Toluolphase mit Wasser gewaschen, über $Na_2SO_4$ getrocknet und im Vakuum eingeengt. Das entstandene Produkt wird durch Kugelrohrdestillation, Umkristallisation oder Säulenchromatographie weiter gereinigt.

Gemäß dieser Vorschrift werden die folgenden Verbindungen erhalten.

| Bsp. Nr. | Het | X | 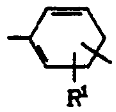 | $\left[O\,(C)_n-(C-C)_o\right]_p$-Y- | Z | Siedep. (°C/Druck) oder Schmp. °C |
|---|---|---|---|---|---|---|
| 97 | 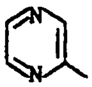 | O | 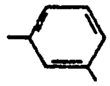 | $-O-CH_2CH_2-O-$ | 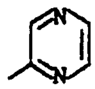 | gelbes Wachs |
| 98 | 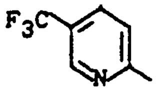 | O | 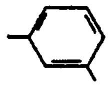 | $-OCH_2CH_2O-$ | 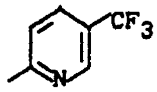 | 230 - 240°C / 0,2 mm |
| 99 | 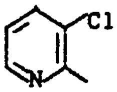 | O | 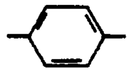 | $-O-CH_2CH_2O-$ | 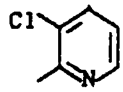 | 210 - 230°C / 0,3 mm 108°C |
| 100 | 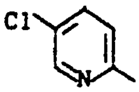 | O | 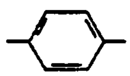 | $-S-CH_2CH_2O-$ | 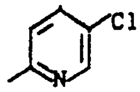 | 215 - 230°C / 0,3 mm |
| 101 | 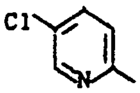 | O | 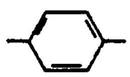 | $-OCH_2CH_2O-$ | 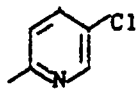 | 220 - 230°C / 0,3 mm |
| 102 | 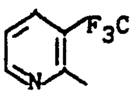 | O | 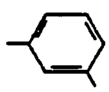 | $-OCH_2CH_2O-$ | 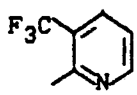 | 230 - 240°C / 0,2 mm |
| 103 | 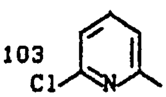 | O | 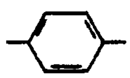 | $-OCH_2CH_2-O-$ | 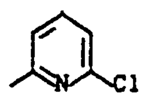 | 230°C / 0,2 mm |
| 104 | 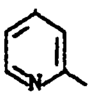 | O | 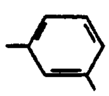 | $-OCH_2CH_2-O-$ | 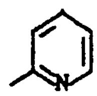 | 220-230°C / 0,4 mm |
| 105 | 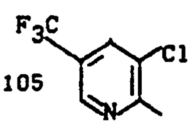 | O | 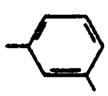 | $-OCH_2CH_2-O-$ | 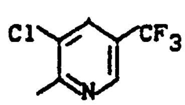 | 240-250°C / 0,1 mm |

| Bsp. Nr. | Het | X | 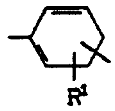 | [O (C)n-(C-C)o]p -Y- | Z | Siedep. (°C/Druck. oder Schmp. °C |
|---|---|---|---|---|---|---|
| 106 | 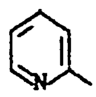 | $CH_2O$ | 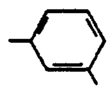 | $-OCH_2CH_2O-CH_2-$ | 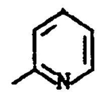 | 230° C / 0,3 mm |
| 107 | 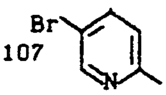 | O | 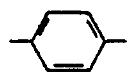 | $-OCH_2CH_2-O-$ | 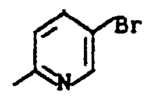 | 230° C / 0,2 mm |
| 108 | 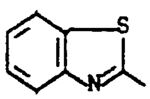 | O | 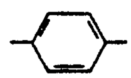 | $-O-CH_2CH_2-O-$ | 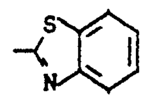 | 250° C/ 0,1 mm |

## Ansprüche

1. Neue substituierte Heteroarylphenylether der Formel (I)

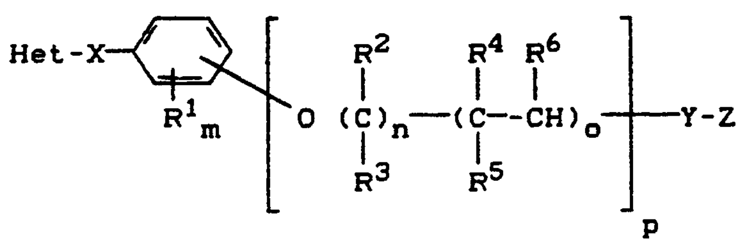

(I)

in welcher
Het für einen gegebenenfalls substituierten heteroaromatischen Rest steht,
X für O, S, $-CH_2-$, $-O-CH_2-$, $-S-CH_2-$, $-CH_2-O-$,

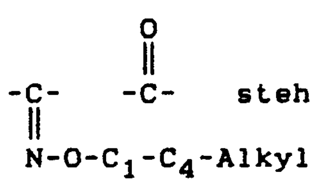

steht;

Y für O, $-O-CH_2-$, S steht,
Z für einen gegebenenfalls substituierten aromatischen oder heteroaromatischen Rest steht;
m für ganze Zahlen von 1 bis 4 steht
n für 0, 1, 2, 3, 4 steht,
o für 0, 1, 2, 3, 4 steht,
wobei n und o nicht gleichzeitig für 0 stehen dürfen,
p für ganze Zahlen von 1 bis 4 steht,
$R^2$, $R^3$, $R^4$, $R^5$, $R^6$ unabhängig voneinander für Wasserstoff, $C_1$-$C_6$-Alkyl, das gegebenenfalls durch Halogen oder $C_{1-4}$-Alkoxy substituiert ist, stehen; zwei benachbart voneinander stehende Reste können auch gemeinsam mit den C-Atomen, an die sie gebunden sind, einen gesättigten carbocyclischen 5- oder 6-Ring bilden,

$R^1$ für gleiche oder verschiedene Reste aus der Gruppe Wasserstoff, Halogen, CN, $C_{1-4}$-Alkyl, 1-5-Halogen-$C_{1-4}$-alkyl, $C_{1-4}$-Alkoxy, $C_{1-4}$-Alkylthio, 1-5-Halogen-$C_{1-4}$-alkoxy, 1-5-Halogen-$C_{1-4}$-alkylthio, Phenyl, Phenoxy steht,
wobei die Verbindungen der Formel I gegebenenfalls in Form ihrer Enantiomere, Racemate oder Diastereomere vorliegen können.

2. Verfahren zur Herstellung der substituierten Heteroarylphenylether der Formel (I)

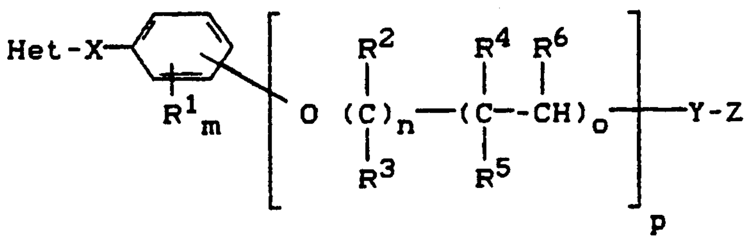

(I)

in welcher
Het für einen gegebenenfalls substituierten heteroaromatischen Rest steht,
X für O, S, $-CH_2-$, $-O-CH_2-$, $-S-CH_2-$, $-CH_2-O-$,

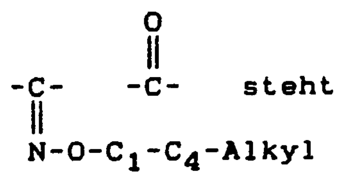

steht;

Y für O, $-O-CH_2-$, S steht,
Z für einen gegebenenfalls substituierten aromatischen oder heteroaromatischen Rest steht;
m für ganze Zahlen von 1 bis 4 steht
n für 0, 1, 2, 3, 4 steht,
o für 0, 1, 2, 3, 4 steht,
wobei n und o nicht gleichzeitig für 0 stehen dürfen,
p für ganze Zahlen von 1 bis 4 steht,
$R^2$, $R^3$, $R^4$, $R^5$, $R^6$ unabhängig voneinander für Wasserstoff, $C_1$-$C_6$-Alkyl, das gegebenenfalls durch Halogen oder $C_{1-4}$-Alkoxy substituiert ist, stehen; zwei benachbart voneinander stehende Reste können auch gemeinsam mit den C-Atomen, an die sie gebunden sind, einen gesättigten carbocyclischen 5- oder 6-Ring bilden,
$R^1$ für gleiche oder verschiedene Reste aus der Gruppe Wasserstoff, Halogen, CN, $C_{1-4}$-Alkyl, 1-5-Halogen-$C_{1-4}$-alkyl, $C_{1-4}$-Alkoxy, $C_{1-4}$-Alkylthio, 1-5-Halogen-$C_{1-4}$-alkoxy, 1-5-Halogen-$C_{1-4}$-alkylthio, Phenyl, Phenoxy steht,
dadurch gekennzeichnet, daß man

a) Heteroarylphenolether der Formel (II)

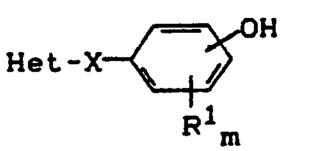

(II)

in welcher
Het, X, $R^1$, m die oben angegebene Bedeutung haben,
mit Verbindungen der Formel (III)

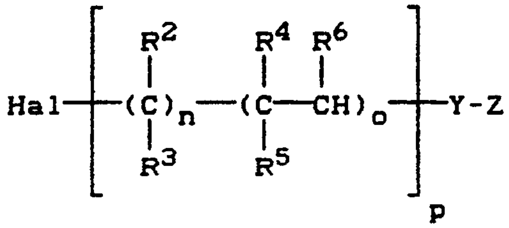

(III)

in welcher
Hal für Halogen steht und
Y, Z, $R^2$ - $R^6$, n, o, p die oben angegebene Bedeutung haben,
umsetzt oder

b) Verbindungen der Formel (IV)

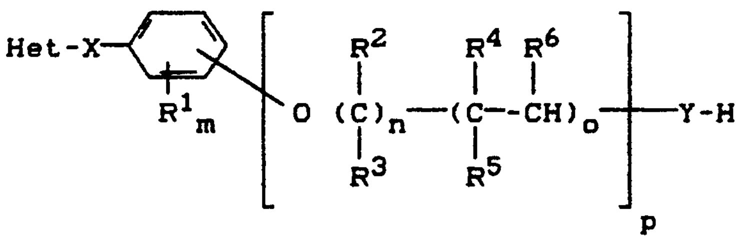

(IV)

in welcher
X, Het, $R^1$ - $R^6$, m, n, o, p die oben angegebene Bedeutung haben und
Y für O oder S steht,
mit Verbindungen der Formel (V)
Hal-Z oder Hal-$CH_2$-Z (V)
in welcher
Hal für Halogen steht
Z die oben angegebene Bedeutung hat,
umsetzt oder

c) Verbindungen der Formel (VI)

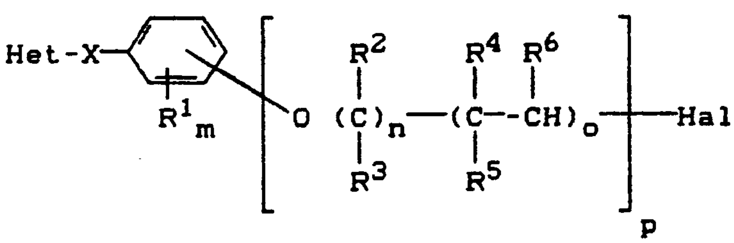

(VI)

in welcher
Hal für Halogen steht,
X, Hat, $R^1$ - $R^6$, m, n, o, p die oben angegebene Bedeutung haben,
mit Verbindungen der Formel (VII)
H-Y-Z (VII)
in welcher
Y und Z die oben angegebene Bedeutung haben,
umsetzt oder

d) für den Fall, daß Het und Z für identische Heterocyclen stehen, man Verbindungen der Formel (VIII)

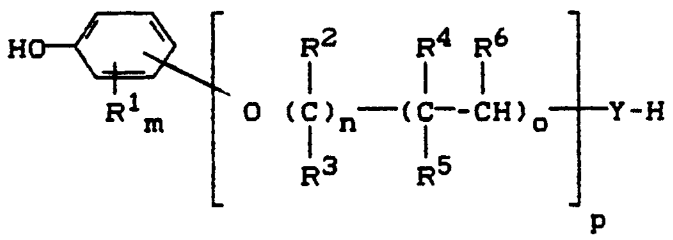

(VIII)

in welcher
$R^1$ - $R^6$, m, n, o, p die oben angegebene Bedeutung haben und
Y für O oder S steht,
mit heterocyclischen Verbindungen der Formel (IX)
Het-Hal oder Het-$CH_2$-Hal (IX)
in welcher
Hal für Halogen steht,
Het die oben angegebene Bedeutung hat,
umsetzt.

3. Verwendung von substituierten Heteroarylphenylethern der allgemeinen Formel I zur Bekämpfung von Insekten, insbesondere von Flöhen.

4. Mittel zur Bekämpfung von Insekten, insbesondere von Flöhen, gekennzeichnet durch einen Gehalt an mindestens einem substituierten Heteroarylphenylether der Formel I, gemäß Anspruch 1.

5. Verfahren zur Bekämpfung von Insekten, insbesondere von Flöhen, dadurch gekennzeichnet, daß man substituierte Heteroarylphenylether der Formel I gemäß Anspruch 1 auf Insekten, ihre Wirtstiere und/oder ihren Lebensraum einwirken läßt.

6. Verfahren zur Herstellung von Mitteln zur Bekämpfung von Insekten, insbesondere von Flöhen, dadurch gekennzeichnet, daß man substituierte Heteroarylphenylether der Formel I gemäß Anspruch 1 mit Streckmitteln und/oder oberflächenaktiven Mitteln vermischt.

7. Verwendung von substituierten Heteroarylphenylethern der Formel I gemäß Anspruch 1 zur Herstellung von Bekämpfungsmitteln gegen Insekten, insbesondere Flöhe.

Europäisches Patentamt

# EUROPÄISCHER RECHERCHENBERICHT

Nummer der Anmeldung

EP 90 10 4059

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | EP-A-0 128 648 (SUMITOMO CHEMICAL CO. LTD.)<br>* Ansprüche 1,2,6,8,9 *<br>--- | 1-7 | C 07 D 213/65<br>C 07 D 417/12<br>C 07 D 213/73<br>C 07 D 213/64<br>C 07 D 241/18<br>C 07 D 277/68<br>C 07 D 239/34<br>C 07 D 401/12<br>C 07 D 213/80<br>C 07 D 307/81<br>C 07 D 409/12 //<br>C 07 D 405/12<br>C 07 D 213/68<br>C 07 D 213/70<br>C 07 D 213/30<br>A 01 N 43/40<br>A 01 N 43/54<br>-/- |
| A | EP-A-0 255 935 (NIPPON KAJAKU K.K.)<br>* Beispiele 95-97; Ansprüche *<br>--- | 1-7 | |
| A | EP-A-0 218 543 (SANDOZ AG et al.)<br>* Ansprüche *<br>--- | 1-7 | |
| A,D | DE-A-2 520 145 (CIBA-GEIGY AG)<br>* Ansprüche *<br>--- | 1-7 | |
| A | DE-A-2 520 177 (CIBA-GEIGY AG)<br>* Ansprüche *<br>--- | 1-7 | |
| A | PATENT ABSTRACTS OF JAPAN<br>Band 9, Nr. 96, 25. April 1985, (C-278)(1819); & JP-A-59227861 (MITSUI TOATSU KAGAKU K.K.) 21.12.1984<br>----- | 1-7 | |

RECHERCHIERTE SACHGEBIETE (Int. Cl.5)

C 07 D 213/00
C 07 D 417/00
C 07 D 241/00
C 07 D 277/00
C 07 D 239/00
C 07 D 401/00
C 07 D 307/00
C 07 D 409/00
C 07 D 405/00
A 01 N 43/00

Der vorliegende Recherchenbericht wurde für alle Patentansprüche erstellt

| Recherchenort | Abschlußdatum der Recherche | Prüfer |
|---|---|---|
| BERLIN | 25-06-1990 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 90 10 4059

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
| | | | A 01 N 43/60<br>A 01 N 43/74<br>A 01 N 43/12 |
| | | | RECHERCHIERTE SACHGEBIETE (Int. Cl.5) |
| | | | |

Der vorliegende Recherchenbericht wurde für alle Patentansprüche erstellt

| Recherchenort | Abschlußdatum der Recherche | Prüfer |
|---|---|---|
| BERLIN | 25-06-1990 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)